# EUROPEAN PATENT APPLICATION

(11) **EP 2 927 327 A1**
(43) Date of publication of application: **07.10.2015**
(21) Application number: 14163121.8
(22) Date of filing: 02.04.2014
(51) Int. Cl.: C12Q 1/68

(54) **Non-invasive in vitro method for diagnosis and prognosis of colorectal cancer**

(71) Applicant: EntreChem, S.L., 33006 Oviedo Asturias (ES)
(72) Inventor: Costales Vigil, Paula, 33006 Oviedo (ES); Ocón Barbas, Santiago, 33006 Oviedo (ES); Morís Varas, Francisco, 33006 Oviedo (ES); Rodrigo Sáez, Luis, 33006 Oviedo (ES); Collado Amores, María Carmen, 46980 Valencia (ES); Mira Obrador, Alejandro, 46020 Valencia (ES)
(74) Representative: Illescas, Manuel

(57) **Abstract**

The present invention belongs to the field of diagnosis and/or prognosis of colorectal cancer. Thus the present invention is focused on a method and kit for the *in vitro* diagnosis and/or prognosis of the colorectal cancer process. More specifically, the present invention refers to the identification of specific microbiota composition in a fecal sample, obtained from a subject suffering colorectal cancer, which can be used as biomarker of colorectal cancer progression.

SEQUENCE LISTING

<110> EntreChem, S.L.

<120> NON-INVASIVE IN VITRO METHOD FOR DIAGNOSIS AND PROGNOSIS OF COLORECTAL
CANCER

<130> EP-06713

<160> 55

<170> BiSSAP 1.3

<210> 1
<211> 60
<212> DNA
<213> Artificial sequence

<220>
<223> Primer Bacteria <prokaryote>

<400> 1
ccatctcatc cctgcgtgtc tccgactcag aaccaacctc agagtttgat cmtggctcag     60

<210> 2
<211> 60
<212> DNA
<213> Artificial sequence

<220>
<223> Primer Bacteria <prokaryote>

<400> 2
ccatctcatc cctgcgtgtc tccgactcag aaccggaatc agagtttgat cmtggctcag     60

<210> 3
<211> 60
<212> DNA
<213> Artificial sequence

<220>
<223> Primer Bacteria <prokaryote>

<400> 3
ccatctcatc cctgcgtgtc tccgactcag aaccttcctc agagtttgat cmtggctcag     60

<210> 4
<211> 60
<212> DNA
<213> Artificial sequence

<220>
<223> Primer Bacteria <prokaryote>

<400> 4
ccatctcatc cctgcgtgtc tccgactcag aagccgaatc agagtttgat cmtggctcag     60

<210> 5

<211> 60
<212> DNA
<213> Artificial sequence

<220>
<223> Primer Bacteria <prokaryote>

<400> 5
ccatctcatc cctgcgtgtc tccgactcag aagcggattc agagtttgat cmtggctcag     60


<210> 6
<211> 60
<212> DNA
<213> Artificial sequence

<220>
<223> Primer Bacteria <prokaryote>

<400> 6
ccatctcatc cctgcgtgtc tccgactcag aaggttcctc agagtttgat cmtggctcag     60


<210> 7
<211> 60
<212> DNA
<213> Bacteria <prokaryote>

<400> 7
ccatctcatc cctgcgtgtc tccgactcag aatacgcctc agagtttgat cmtggctcag     60


<210> 8
<211> 60
<212> DNA
<213> Artificial sequence

<220>
<223> Primer Bacteria <prokaryote>

<400> 8
ccatctcatc cctgcgtgtc tccgactcag acaccacatc agagtttgat cmtggctcag     60


<210> 9
<211> 60
<212> DNA
<213> Artificial sequence

<220>
<223> Primer Bacteria <prokaryote>

<400> 9
ccatctcatc cctgcgtgtc tccgactcag acatctcttc agagtttgat cmtggctcag     60


<210> 10
<211> 60
<212> DNA
<213> Artificial sequence

<220>
<223> Primer Bacteria <prokaryote>

<400> 10
ccatctcatc cctgcgtgtc tccgactcag acagactctc agagtttgat cmtggctcag     60


<210> 11
<211> 60
<212> DNA
<213> Artificial sequence

<220>
<223> Primer Bacteria <prokaryote>

<400> 11
ccatctcatc cctgcgtgtc tccgactcag accaaccatc agagtttgat cmtggctcag     60


<210> 12
<211> 60
<212> DNA
<213> Artificial sequence

<220>
<223> Primer Bacteria <prokaryote>

<400> 12
ccatctcatc cctgcgtgtc tccgactcag accaagcttc agagtttgat cmtggctcag     60


<210> 13
<211> 60
<212> DNA
<213> Artificial sequence

<220>
<223> Primer Bacteria <prokaryote>

<400> 13
ccatctcatc cctgcgtgtc tccgactcag acctaggttc agagtttgat cmtggctcag     60


<210> 14
<211> 60
<212> DNA
<213> Artificial sequence

<220>
<223> Primer Bacteria <prokaryote>

<400> 14
ccatctcatc cctgcgtgtc tccgactcag accttccatc agagtttgat cmtggctcag     60


<210> 15
<211> 60
<212> DNA
<213> Artificial sequence

<220>
<223> Primer Bacteria <prokaryote>

<400> 15
ccatctcatc cctgcgtgtc tccgactcag acgaagcatc agagtttgat cmtggctcag     60

<210> 16
<211> 60
<212> DNA
<213> Artificial sequence

<220>
<223> Primer Bacteria <prokaryote>

<400> 16
ccatctcatc cctgcgtgtc tccgactcag actcgacatc agagtttgat cmtggctcag     60


<210> 17
<211> 60
<212> DNA
<213> Artificial sequence

<220>
<223> Primer Bacteria <prokaryote>

<400> 17
ccatctcatc cctgcgtgtc tccgactcag actcgagttc agagtttgat cmtggctcag     60


<210> 18
<211> 60
<212> DNA
<213> Artificial sequence

<220>
<223> Primer Bacteria <prokaryote>

<400> 18
ccatctcatc cctgcgtgtc tccgactcag actgacactc agagtttgat cmtggctcag     60


<210> 19
<211> 60
<212> DNA
<213> Artificial sequence

<220>
<223> Primer Bacteria <prokaryote>

<400> 19
ccatctcatc cctgcgtgtc tccgactcag agacgacatc agagtttgat cmtggctcag     60


<210> 20
<211> 60
<212> DNA
<213> Artificial sequence

<220>
<223> Primer Bacteria <prokaryote>

<400> 20
ccatctcatc cctgcgtgtc tccgactcag agagtctctc agagtttgat cmtggctcag     60


<210> 21

<211> 52
<212> DNA
<213> Artificial sequence

<220>
<223> Primer Bacteria <prokaryote>

<400> 21
cctatcccct gtgtgccttg gcagtctcag gcttaccgcg gckgctggca cg          52


<210> 22
<211> 22
<212> DNA
<213> Artificial sequence

<220>
<223> Primer Bifidobacterium


<400> 22
gattctggct caggatgaac gc                                           22


<210> 23
<211> 20
<212> DNA
<213> Artificial sequence

<220>
<223> Primer Bifidobacterium


<400> 23
ctgataggac gcgaccccat                                              20


<210> 24
<211> 20
<212> DNA
<213> Artificial sequence

<220>
<223> Primer Blautia coccoides


<400> 24
aaatgacggt acctgactaa                                              20


<210> 25
<211> 22
<212> DNA
<213> Artificial sequence

<220>
<223> Primer Blautia coccoides

<400> 25
ctttgagttt cattcttgcg aa                                           22


<210> 26

<211> 20
<212> DNA
<213> Artificial sequence

<220>
<223> Primer Bacteroides-Prevotella


<400> 26
ggtgtcggct taagtgccat                                                   20


<210> 27
<211> 18
<212> DNA
<213> Artificial sequence

<220>
<223> Primer Bacteroides-Prevotella

<400> 27
cggaygtaag ggccgtgc                                                     18


<210> 28
<211> 19
<212> DNA
<213> Artificial sequence

<220>
<223> Primer Lactobacillus


<400> 28
agcagtaggg aatcttcca                                                    19


<210> 29
<211> 17
<212> DNA
<213> Artificial sequence

<220>
<223> Primer Lactobacillus

<400> 29
caccgctaca catggag                                                      17


<210> 30
<211> 20
<212> DNA
<213> Artificial sequence

<220>
<223> Primer Akkermansia muciniphila


<400> 30
cagcacgtga aggtggggac                                                   20


<210> 31

<211> 20
<212> DNA
<213> Artificial sequence

<220>
<223> Primer Akkermansia muciniphila

<400> 31
ccttgcggtt ggcttcagat                                                    20


<210> 32
<211> 25
<212> DNA
<213> Artificial sequence

<220>
<223> Primer Enterobacteriaceae


<400> 32
cattgacgtt acccgcagaa gaagc                                              25


<210> 33
<211> 21
<212> DNA
<213> Artificial sequence

<220>
<223> Primer Enterobacteriaceae

<400> 33
ctctacgaga ctcaagcttg c                                                  21


<210> 34
<211> 17
<212> DNA
<213> Artificial sequence

<220>
<223> Primer Bacteria <prokaryote>

<400> 34
cgtgccagca gccgcgg                                                       17


<210> 35
<211> 25
<212> DNA
<213> Artificial sequence

<220>
<223> Primer Bacteria <prokaryote>

<400> 35
tggactacca gggtatctaa tcctg                                              25


<210> 36
<211> 24
<212> DNA

<213> Artificial sequence

<220>
<223> Primer Faecalibacterium prausnitzii


<400> 36
ccatgaattg ccttcaaaac tgtt                                              24


<210> 37
<211> 20
<212> DNA
<213> Artificial sequence

<220>
<223> Primer Faecalibacterium prausnitzii

<400> 37
gagcctcagc gtcagttggt                                                   20


<210> 38
<211> 29
<212> DNA
<213> Artificial sequence

<220>
<223> Primer Fusobacterium nucleatum


<400> 38
caaccattac tttaactcta ccatgttca                                         29


<210> 39
<211> 33
<212> DNA
<213> Artificial sequence

<220>
<223> Primer Fusobacterium nucleatum

<400> 39
gttgacttta cagaaggaga ttatgtaaaa atc                                     33


<210> 40
<211> 19
<212> DNA
<213> Artificial sequence

<220>
<223> Primer Methanobacteriales


<400> 40
cgwagggaag ctgttaagt                                                    19


<210> 41
<211> 18
<212> DNA

<213> Artificial sequence

<220>
<223> Primer Methanobacteriales

<400> 41
agcaccacaa cgcgtgga                                                    18


<210> 42
<211> 18
<212> DNA
<213> Artificial sequence

<220>
<223> Primer Methanobacteriales

<400> 42
taccgtcgtc cactcctt                                                    18


<210> 43
<211> 18
<212> DNA
<213> Artificial sequence

<220>
<223> Primer Methanomicrobiales


<400> 43
atcgrtacgg gttgtggg                                                    18


<210> 44
<211> 24
<212> DNA
<213> Artificial sequence

<220>
<223> Primer Methanomicrobiales


<400> 44
tycgacagtg aggracgaaa gctg                                             24


<210> 45
<211> 21
<212> DNA
<213> Artificial sequence

<220>
<223> Primer Methanomicrobiales


<400> 45
cacctaacgc rcathgttta c                                                21


<210> 46
<211> 18
<212> DNA

<213> Artificial sequence

<220>
<223> Primer Methanosarcinaceae


<400> 46
gaaaccgyga taagggga                                                    18


<210> 47
<211> 18
<212> DNA
<213> Artificial sequence

<220>
<223> Primer Methanosarcinaceae

<400> 47
ttagcaaggg ccgggcaa                                                    18


<210> 48
<211> 18
<212> DNA
<213> Artificial sequence

<220>
<223> Primer Methanosarcinaceae

<400> 48
tagcgarcat cgtttacg                                                    18


<210> 49
<211> 20
<212> DNA
<213> Artificial sequence

<220>
<223> Primer Methanosarcinaceae

<400> 49
taatcctyga rggaccacca                                                  20


<210> 50
<211> 22
<212> DNA
<213> Artificial sequence

<220>
<223> Primer Methanosarcinaceae

<400> 50
acggcaaggg acgaaagcta gg                                               22


<210> 51
<211> 17
<212> DNA
<213> Artificial sequence

<220>
<223> Primer Methanosarcinaceae

<400> 51
cctacggcac cracmac                                                    17


<210> 52
<211> 22
<212> DNA
<213> Artificial sequence

<220>
<223> Primer Methanobrevibacter


<400> 52
tgggaaactg ggataatac tg                                               22


<210> 53
<211> 22
<212> DNA
<213> Artificial sequence

<220>
<223> Primer Methanobrevibacter

<400> 53
aatgaaaagc catcccgtta ag                                              22


<210> 54
<211> 21
<212> DNA
<213> Artificial sequence

<220>
<223> Primer Methanobrevibacter smithii


<400> 54
aacagaaaac ccagtgaaga g                                               21


<210> 55
<211> 21
<212> DNA
<213> Artificial sequence

<220>
<223> Primer Methanobrevibacter smith

<400> 55
acgtaaaggc actgaaaaac c                                               21

## Description

### FIELD OF THE INVENTION

The present invention may be included in the field of the medicine in general, more particularly in the field of diagnosis and/or prognosis of colorectal cancer.

Thus the present invention is focused on a method and kit for the *in vitro* diagnosis and/or prognosis of colorectal cancer. More specifically, the present invention refers to the identification of specific microbiota composition in a fecal sample, obtained from a subject suffering from colorectal cancer and/or colorectalpolyps, which can be used as biomarker of colorectal cancer progression.

### STATE OF THE ART

Colorectal cancer (CRC) is the most common malignancy in the Western world as it is the second most prevalent tumor in men (after lung cancer) and in women (followed by breast cancer). It also constitutes the second most common cause of cancer death, which is about 50% (Cunningham D., et al. Lancet. 2010;375:1030-47).
Although CRC etiology remains unknown, environmental factors including dietary habits can influence the disease progression as well as endogenous factors, such as changes in the intestinal microbiota (Bamia C., et al. Eur. J. Epidemiol. 2013; 28:317-28; Sobhani I., et al. Therap. Adv. Gastroenterol. 2013;6:215-29). There are certain special risk groups, including Lynch syndrome, familial adenomatous polyposis, family and personal history of CRC, some cases of chronic inflammatory bowel disease, presence of adenomatous colon polyps and age over 50 years.

Within the human colon up to one hundred trillion bacteria coexist in commensal balance in healthy individuals, playing a crucial role in food digestion, protection of intestinal mucosa, and modulation of the immune system. It has been suggested that environmental cancer risk is determined by the interaction between diet and colonic microbial metabolism. Because of the preponderant role of intestinal microbiota in gut development and pathology, especially inflammatory bowel disease (IBD) as a risk factor for CRC, early efforts have focused on elucidating the multifactorial role of gut microbes in CRC. Recently, many efforts have been made to unveil the contribution of the intestinal microbiota to gut diseases, employing culture-independent techniques. Alterations in microbial community structure influence the health condition of the intestine as shown in both human and animal models (Regula J., et al. N. Engl. J. Med. 2006; 355:1863-72; Giardiello F.M., et al. Gastroenterology 2001;21:198-213; Bamia C., et al. Eur. J. Epidemiol. 2013; 28:317-28).

There is evidence of microbial dysbiosis in colorectal cancer patients, particularly when comparing between CRC tissue and adjacent non-malignant mucosa as well as in inflammation-induced tumor progression of some polyps to cancer. Sobhani and Tap described an increase in Bacteroides-Prevotella group in CRC mucosa [Sobhani I., et al. PLoS One. 2011 Jan 27;6(1)], whereas Chen and coworkers identified shifts involving more than one genus in cancer patients [Chen W., et al. PLoS One. 2012;7(6)]. Differences in fecal microbiota have also been reported between CRC and patients with colonic polyps compared to controls, with a significant increase of two Clostridium species (Scanlan P.D., et al. Environmental Microbiol. 2008;10:789-98).

In contrast to gastric cancer, where just one bacteria (*Helicobacter pylori)* has been associated to the disease, many pathogenic species have been assessed as directly responsible in 15% of the total colon cancer cases and among them, *Fusobacterium nucleatum* has been found strongly prevalent. *Akkermansia muciniphila,* a mucin-degrading bacterium might also be involved in the formation of an inflammatory-driven colonic disease. Additionally, little is known about the relationship between archaea methanogens species and different colonic diseases, particularly with CRC.

Once CRC has been diagnosed, treatment decisions are typically made using the stage of cancer progression. A number of techniques are employed to stage the cancer (some of which are also used to screen for colon cancer), including pathologic examination of resected colon, sigmoidoscopy, colonoscopy, and various imaging techniques. Proximal lymph node evaluation, sentinel node evaluation, chest/abdominal/pelvic CT, MRI scans, positron emission tomography ("PET") scans, liver functionality tests (for liver metastases), and blood tests (complete blood count ("CBC"), carcinoembryonic antigen ("CEA"), CA 19-9) are employed to determine the stage. Several classification systems have been devised to stage the extent of CRC, including the Dukes' system and the more detailed International Union against Cancer- American Joint Committee on Cancer TNM staging system.

The TNM system, which is used for either clinical or pathological staging, is divided into four stages, each of which evaluates the extent of cancer growth with respect to primary tumor (T), regional lymph nodes (N), and distant metastasis (M). The system focuses on the extent of tumor invasion into the intestinal wall; invasion of adjacent structures; the number of regional lymph nodes that have been affected; and whether distant metastasis has occurred. Stage 0 is characterized by in situ carcinoma (Tis), in which the cancer cells are located inside the glandular basement membrane (intraepithelial) or lamina propria (intramucosal). In this stage, the cancer has not spread to the regional lymph nodes (NO), and there is no distant metastasis (MO). In stage I, there is still no spread of the cancer to the regional lymph nodes and no distant metastasis, but the tumor has invaded the submucosa (TI) or has progressed further to invade the muscularis propria (T2). Stage II also involves no spread of the cancer to the regional lymph nodes and no distant metastasis, but the tumor has invaded the subserosa, or the nonperitonealized pericolic or perirectal tissues (T3), or has progressed to invade other organs or structures, and/or has perforated the visceral peritoneum (T4). Stage III is characterized by any of the T substages, no distant metastasis, and either spread to 1 to 3 regional lymph nodes (NI) or spread to four or more regional lymph nodes (N2). Lastly, stage IV involves any of the T or N substages, as well as distant metastasis. Physicians will also assign a grade, that is, characterize CRC based on the appearance of the cells ranging from GI (well-differentiated, almost normal) to G4 (undifferentiated, very abnormal) where a high grade is an indication of a poor prognosis.

For the treatment of CRC, surgical resection results in a cure for roughly 50% of patients. Chemotherapy and irradiation maybe used both preoperatively (neoadjuvant) and postoperatively (adjuvant) in treating CRC. Chemotherapeutic agents, particularly 5-fluorouracil (5-FU), are powerful weapons in treating CRC. Other agents include oxaliplatin (Eloxatin(R)), irinotecan (Camptosar(R)), leucovorin, capecitabine (Xeloda(R)), bevacizumab (Avastin(R)), cetuximab (Erbitux(R)), and panitumumab (Vectibix(R)). These drugs are frequently combined. Common combinations are FOLFOX (5-FU, leucovorin, oxaliplatin); FOLFIRI (5-FU, leucovorin, irinotecan); and FOLFOXIRI (5-FU, leucovorin, irinotecan, oxaliplatin). Bevacizumab is a targeted therapeutic, specifically a monoclonal antibody that binds to vascular endothelial growth factor (VEGF) to prevent formation of blood vessels around the tumor. Cetuximab and panitumumab are monoclonal antibodies that target epidermal growth factor receptor (EGFR).

Many patients will develop a recurrence of CRC following surgical resection, particularly in the first 2 or 3 years. Accordingly, CRC patients must be closely monitored to determine response to therapy and to detect persistent or recurrent disease and metastasis.

Because CRC is often treatable when detected at an early, localized stage, current guidelines recommend screening tests should be a part of routine care for all adults starting at age 50. The current tests may be divided into two types: fecal tests (non-invasive) and structural examination tests (invasive). Examples of fecal tests are (i) the fecal occult blood test ("FOBT"); (ii) the fecal immunochemical test ("FIT"); and (iii) the stool DNA ("sDNA") test. Structural examination tests are (i) colonoscopy; (ii) flexible sigmoidoscopy; (iii) double-contrast barium enema ("DCBE"); (iv) CT colonography (virtual colonoscopy); and (v) capsule endoscopy. These tests have advantages and disadvantages. Current fecal tests suffer from issues of accuracy, precision, inter- and intra- individual variability, and compliance due to patients being uncomfortable with sample collection. If a fecal test is positive, a patient will be referred for a colonoscopy for a thorough examination and intervention (removal of adenomas) if necessary. The structural examination tests require both purging of a patient's bowels and pumping air into the colon to aid visualization.

The fecal blood tests screen for CRC by detecting the amount of blood in the stool. The tests are based on the premise that neoplastic tissue, particularly malignant tissue, bleeds more than typical mucosa, with the amount of bleeding increasing with polyp size and cancer stage. Multiple testing is recommended because of intermittent bleeding. While fecal blood tests may detect some early stage tumors in the lower colon, they are unable to detect (i) CRC in the upper colon because any blood will be metabolized and/or (ii) smaller adenomatous polyps, thus creating false negatives. Any gastro-intestinal bleeding due to haemorrhoids, fissures, inflammatory disorders (ulcerative colitis, Crohn's disease), infectious diseases, even long distance running, will create false positives (Beg et al. J. Indian Acad. Clin. Med. 2002; 3(2) 153-158).

The sDNA test measures a variety of DNA markers measured in a lab from a stool sample collected by the patient. Current sDNA tests, available from Exact Sciences Corp. (Madison, WI), measure mutations in K-ras, APC, P53 genes; BAT -26 (an MSI marker); a marker for DNA integrity; and methylation of the vimentin gene (Levin et al. CA. Cancer J. Clinicians. 2008; 58(3):130-160). While some guidelines recommend sDNA testing other guidelines are more conservative and do not recommend sDNA testing.

The invasive techniques such as colonoscopy, sigmoidoscopy, double-contrast barium enema, etc., allows direct visualization of the bowel, and enables one to detect, biopsy, and remove adenomatous polyps. Colonoscopy is the "gold standard" diagnostic for colon cancer but have downsides. Colonoscopy is a relatively expensive procedure and there are risks of possible bowel perforation and haemorrhaging. Moreover, the skill and experience of doctors vary and some studies have reported missing 6-12% of large adenomas (=10 mm) and failing to detect cancer in 5% of the cases.

It is clear that improved procedures used for detecting, diagnosing, monitoring, staging, prognosticating, and preventing the recurrence of CRC are of critical importance to the outcome of the patient. Moreover, current procedures, while helpful in each of these analyses, are limited by their specificity, sensitivity, invasiveness, and/or cost effectiveness. As such, minimally invasive, highly specific and sensitive procedures would be highly desirable. Accordingly, there is a great need for more sensitive, accurate and non-invasive methods for predicting whether a person is likely to develop CRC, for diagnosing CRC and for monitoring the progression of the disease.

The solution proposed by the present invention is the identification of specific microbiota, in a biological sample from a subject, which are able to monitor the progression of the disease and also classify a CRC patient into a group with poor or favourable prognosis.

### DESCRIPTION OF THE INVENTION

The present invention is focused on the evaluation and assessment of the microbial composition, preferably Bacteria and Archaea, in biological samples selected from: tumors, colonic polyps, fecal samples and healthy sites, in order to define a specific group of bacteria consortia which serve as bacterial biomarkers of diagnosis of colorectal cancer and also as biomarkers in the colorectal cancer progression with better efficiency than classical or conventional methods.

In particular non-limiting embodiments, this disclosure is directed to an *in vitro* method and a kit for carrying out the same, for diagnosis and/or prognosis of colorectal cancer in a subject, using a biological sample obtained from said subject which comprises:
(a) measuring a level of Archaea microorganism in said biological sample, wherein said Archaea microorganism is a methanogen; and
(b) comparing the level of Archaea microorganism measured in (a) with a reference level of Archaea microorganism in at least one control sample, wherein an elevated level is indicative of colorectal cancer in said subject.

In another embodiment of the invention, an *in vitro* method for diagnosis and/or prognosis of colorectal cancer in a subject, using a biological sample obtained from said subject is also disclosed, which comprises:
(a) measuring the level of *Akkermansia muciniphila* in the biological sample, and
(b) comparing the level of of *Akkermansia muciniphila* measured in (a) with a reference level of *Akkermansia muciniphila* in at least one control sample, wherein a reduced level of *Akkermansia muciniphila* is indicative of colorectal cancer in said subject and/or greater severity of colorectal cancer.

Said method may be carried out independently or jointly with the method above disclosed based on the measurement of Archaea microorganisms in a biological sample.

The present invention also discloses a kit for *in vitro* detection of colorectal cancer in a biological sample obtained from a subject which comprises:
a) a means for measuring a level of Archaea microorganism and/or a level of *Akkermansia muciniphila* in said biological sample, wherein said Archaea microorganism is a methanogen; and
b) instructions for comparing the level of Archaea microorganism and/or the level of *Akkermansia muciniphila* in said biological sample with a reference level of Archaea microorganism and/or a reference level of *Akkermansia muciniphila* in at least one control sample, wherein an elevated level of Archaea microorganism in said biological sample is indicative of colorectal cancer in said subject and wherein a reduced level of *Akkermansia muciniphila* is indicative of colorectal cancer in said subject and/or greater severity of colorectal cancer.

The present invention also discloses the use of the aforementioned methods of the invention or use of the aforementioned kits of the invention in the *in vitro* diagnosis and/or prognosis of colorectal cancer.

To facilitate an understanding of the present invention, a number of terms are defined below:
As used herein the term "Archaea" refers to a domain or kingdom of single-celled microorganisms. These microbes are prokaryotes, meaning they have no cell nucleus or any other membrane-bound organelles in their cells. Archaea was classed with the bacteria, the other prokaryotes, as archaebacteria. The Archaea are further divided into five recognized phyla: Crenarchaeota, Euryarchaeota, Korarchaeota, Nanoarchaeota and Thaumarchaeota. The present invention refers preferably to methanogens of these phyla, more preferably to the Euryarchaeota phylum, even more preferably to the Methanobacteriales order and most preferably to the *Methanobrevibacter* genus.

As an alternative to the methods, kits and uses of the present invention as disclosed above or supplementary to them, in which the level of Archaea and/or *Akkermansia muciniphila* is determined, the total level of Bacteria, preferably the level of Enterobacteriaceae family or the level of Gram-negative bacteria, may instead or supplementary, be determined. Alternatively or supplementary, the level of other bacteria may be also determined, including the level of bacteria from the Bifidobacterium genus, the level of bacteria from the *Blautia coccoides* group, the level of *Bacteroides-Prevotella,* the level of *Lactobacillus* spp., the level of *Faecalibacterium prausnitzii,* the level of *Fusobacterium nucleatum.* Preferably the level of *Bacteroides* or *Parabacteroides* is determined instead of the level of *Akkermansia muciniphila.* As used herein, the term *"Bacteroides"* and *"Parabacteroides"* refers to a genus and a species, respectively, of Gram-negative, anaerobic bacteria. Bacteroides spp. are normally mutualistic, making up the most substantial portion of the mammalian gastrointestinal flora.

As used herein, the term "colorectal cancer" includes any type of colon, rectal and appendix neoplasia and refers both to early and late adenomas and to carcinomas as well as to the hereditary, familial or sporadic cancer. Hereditary CRC includes those syndromes which include the presence of polyps, such as the hamartomatous polyposis syndromes and the most known, familial adenomatous polyposis (FAP) as well as nonpolyposis syndromes such as hereditary nonpolyposis colorectal cancer (HNPCC) or Lynch syndrome I. In a preferred embodiment of the invention, said colorectal cancer (CRC) is colon cancer, rectal cancer and/or vermiform appendix cancer. In another embodiment of the invention, said colorectal cancer is stage 0, stage I, stage II, stage III and/or stage IV colorectal cancer, each of which evaluates the extent of cancer growth with respect to primary tumor (T), regional lymph nodes (N), and distant metastasis (M). This classification focuses on the extent of tumor invasion into the intestinal wall; invasion of adjacent structures; the number of regional lymph nodes that have been affected; and whether distant metastasis has occurred. Stage 0 is characterized by *in situ* carcinoma (Tis), in which the cancer cells are located inside the glandular basement membrane (intraepithelial) or lamina propria (intramucosal). In this stage, the cancer has not spread to the regional lymph nodes (NO), and there is no distant metastasis (MO). In stage I, there is still no spread of the cancer to the regional lymph nodes and no distant metastasis, but the tumor has invaded the submucosa (TI) or has progressed further to invade the muscularis propria (T2). Stage II also involves no spread of the cancer to the regional lymph nodes and no distant metastasis, but the tumor has invaded the subserosa, or the nonperitonealized pericolic or perirectal tissues (T3), or has progressed to invade other organs or structures, and/or has perforated the visceral peritoneum (T4). Stage III is characterized by any of the T substages, no distant metastasis, and either spread to 1 to 3 regional lymph nodes (NI) or spread to four or more regional lymph nodes (N2). Lastly, stage IV involves any of the T or N substages, as well as distant metastasis. Physicians will also assign a grade, that is, characterize CRC based on the appearance of the cells ranging from GI (well-differentiated, almost normal) to G4 (undifferentiated, very abnormal) where a high grade is an indication of a poor prognosis. The stages of CRC referred to herein correspond to the American Joint Committee on Cancer (AJCC) CRC staging, although other staging methods, such as Dukes and Astler-Coller staging, can equally be used.

As used herein, the term "subject" refers to any animal (e.g. a mammal), including, but not limited to, humans, non-human primates, rodents, and the like, which is to be the recipient of a particular treatment. Typically, the terms "subject" and "patient" are used interchangeably herein in reference to a human subject.

As used herein, the term "control sample" refers to a sample or standard used for comparison with an experimental sample, such as a tumor sample obtained from a patient with colon cancer. In some embodiments, the control is a sample obtained from a subject not having colon cancer or a non-cancerous tissue sample obtained from a patient diagnosed with colon cancer, such as a non-cancerous tissue sample from the same organ in which the tumor resides (e.g. non-cancerous colon tissue can serve as a control for a colon cancer). In some embodiments, the control is a standard value or a reference level *(i.e.* a previously tested control sample or group of samples that represent baseline or normal values). Controls or standards for comparison to a sample, for the determination of differential concentration or expression, include samples believed to be normal (in that they are not altered for the desired characteristic, for example a sample from a subject who does not have colon cancer) as well as laboratory values, even though possibly arbitrarily set. Laboratory standards and values may be set based on a known or determined population value and can be supplied in the format of a graph or table that permits comparison of measured, experimentally determined values.

As used herein, the term "non-human animals" refers to all non-human animals including, but are not limited to, vertebrates such as rodents, non-human primates, ovines, bovines, ruminants, lagomorphs, porcines, caprines, equines, canines, felines, aves, etc.

The term "adenoma" refers to a growth of epithelial cells of glandular origin which may be benign or malignant. They are also referred to as adenomatous polyps. Adenomas may be pedunculated (large head with a narrow stalk) or sessile (broad based). They may be classified as tubular adenomas, tubulovillous adenomas, villous adenomas, and flat adenomas. The adenoma may be an adenocarcinoma. The adenoma may be an adenoma from a human patient which may be a large adenoma >10cm, a small adenoma < 5 cm, or an adenoma between 0.5 cm and 15 cm in length.

"Primers" as used herein refer to oligonucleotides that can be used in an amplification method, such as a polymerase chain reaction ("PCR"), to amplify a nucleotide sequence based on the polynucleotide sequence corresponding to a particular genomic sequence, e.g. one specific for particular bacteria. At least one of the PCR primers for amplification of a polynucleotide sequence is sequence-specific for the sequence.

The term "template" refers to any nucleic acid molecule that can be used for amplification in the technology. RNA or DNA that is not naturally double stranded can be made into double stranded DNA so as to be used as template DNA. Any double stranded DNA or preparation containing multiple, different double stranded DNA molecules can be used as template DNA to amplify a locus or loci of interest contained in the template DNA.

The term "amplification reaction" as used herein refers to a process for copying nucleic acid one or more times. In embodiments, the method of amplification includes, but is not limited to, polymerase chain reaction, self-sustained sequence reaction, ligase chain reaction, rapid amplification of cDNA ends, polymerase chain reaction and ligase chain reaction, Q-[beta] replicase amplification, strand displacement amplification, rolling circle amplification, or splice overlap extension polymerase chain reaction. In some embodiments, a single molecule of nucleic acid may be amplified.

The biological sample may be from a subject suspected of having adenoma or from a subject diagnosed with CRC. A biological sample may also be a sample from a mucosal surface, such as a fecal or rectal swab sample, a blood and blood fractions or products (e.g. serum, plasma, platelets, red blood cells, white blood cells, circulating tumor cells isolated from blood, free DNA isolated from blood, and the like), sputum, lymph and tongue tissue, cultured cells, e.g. primary cultures, explants, and transformed cells, stool, urine, etc. Preferably, in the present invention the biological sample refers to a fecal sample. A sample is typically obtained from a eukaryotic organism, most preferably a mammal such as a primate e.g. chimpanzee or human; a cow; a horse; a camel; a dog; a cat; a rodent, e.g. guinea pig, rat or mouse; or a rabbit.

### BRIEF DESCRIPTION OF THE FIGURES

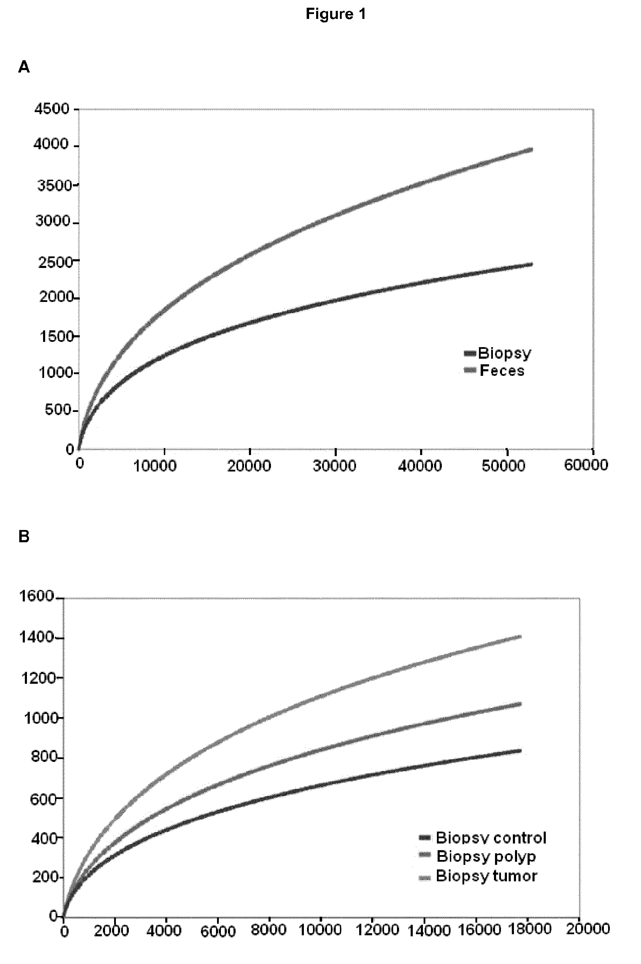

**Figure 1****.** Rarefaction curves.
   Microbial diversity of mucosal tissue (biopsies) and intestinal content (feces). Graphs show rarefaction curves by estimating of the number of bacterial species, as inferred by the number of Operational Taxonomic Units (OTUs). An OTU was a cluster of 16S rRNA sequences which were over 95% identical, a conservative estimate for the boundary between species, established at 97% for full-length 16S gene sequences. Panel A shows rarefaction curves for biopsies and fecal samples. Panel B shows rarefaction curves for the biopsies from the three groups (control, polyp and tumor).
**Figure 2****.** Family (A) and Genus (B).
   Bacterial taxonomic composition of tissues and feces associated to healthy and CRC samples. The graphs show the proportion of bacterial families (Panel A) and genera (Panel B) as inferred by PCR amplification and pyrosequencing of the 16S rRNA.
**Figure 3****.** Principal Coordinates Analyses (PCoA) based on the bacterial composition of samples included in the study.
   Panel A) Distribution of samples (H= correspond to fecal samples; and biopsies are classified as healthy (S), polyp (P) and tumor (T). Panel B) General Distribution of all samples included in the study (F=feces, B= biopsies) which were classified in 3 groups (Control, Polyp and Tumor).
**Figure 4****.** Box and whisker plots of the level of Archaea microorganism in fecal samples of subjects with polyps, subjects with tumors and control subjects.
   Panel A) Level of Methanobacteriales microorganism (log n° gene copies/mg fecal sample). Panel B) Level of Methabrevibacterium (log n° gene copies/mg fecal sample).

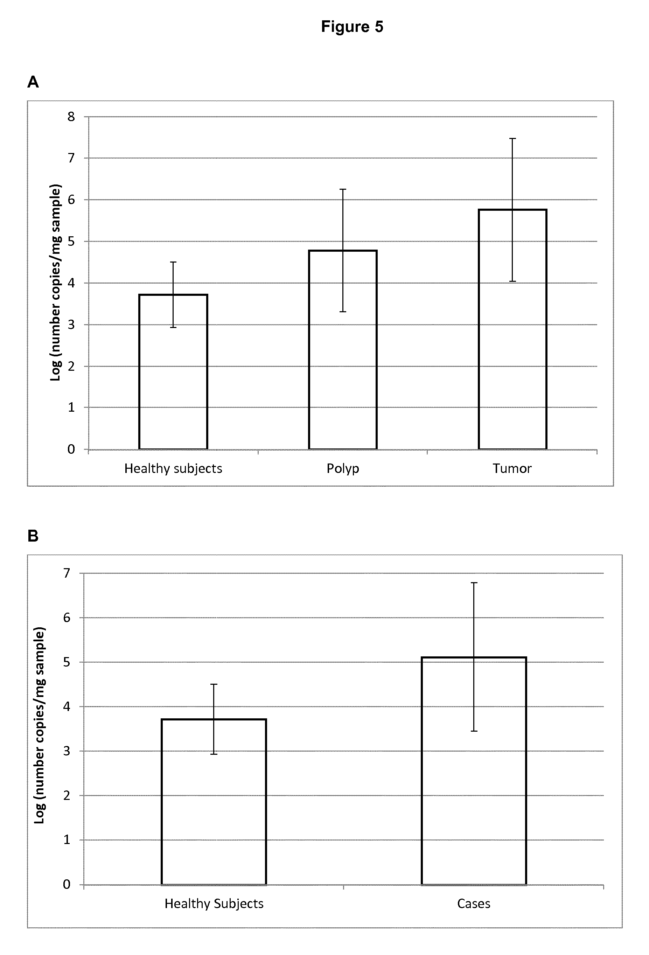

**Figure 5****.** Box and whisker plots of the level of Methanobacteriales microorganism in fecal samples (based on Table 3).
   Panel A) Level of Methanobacteriales microorganism (log n° gene copies/mg fecal sample) in samples from healthy subjects, subjects with polyps and subjects with tumors. Panel B) Level of Methabrevibacterium (log n° gene copies/mg fecal sample) in samples from healthy subjects and diseased subjects (with polyps or tumors).

### Detailed description of the invention

Present invention relates to an *in vitro* method, hereinafter method of the invention, for diagnosis and/or prognosis of colorectal cancer in a subject using a biological sample obtained from said subject which comprises:
a) measuring a level of Archaea microorganism in said biological sample, wherein said Archaea microorganism is a methanogen; and
b) comparing the level of Archaea microorganism measured in (a) with a reference level of Archaea microorganism determined from a control sample or samples, wherein an elevated level is indicative of colorectal cancer in said subject.

The *in vitro* method of the present invention is a method that is not performed upon the subject being tested, but upon a sample which has been obtained from said subject. The method may be carried out in a non-invasive manner, such that it does not require intervention of said subject. The *in vitro* method of the invention is for diagnosis and/or prognosis of colorectal cancer in a subject using a biological sample obtained from said subject. Thus, said method is for diagnosis and/or prognosis of colorectal cancer. Said diagnosis and/or prognosis is performed on a biological sample obtained from a subject. The method of the invention involves a step (a) of measuring a level of Archaea microorganism in said biological sample, wherein said Archaea microorganism is a methanogen. Measuring a level of Archaea microorganism in the biological sample involves determining the quantity of an Archaea methanogen or Archaea methanogens present in said sample, *i.e.* quantification of microbial load in said biological sample. The quantity of an Archaea methanogen or Archaea methanogens present in a biological sample may be determined using a real time quantitative polymerase chain reaction [quantitative polymerase chain reaction (qPCR)] or an immunoassay. In a preferred embodiment of the invention, the level of Archaea microorganism is measured by real time quantitative polymerase chain reaction. Herein, the level of various microorganisms is measured by real time quantitative polymerase chain reaction. The real time quantitative polymerase chain reaction quantifies the microbial makeup of the biological sample based on the number of copies of genes specific to each microorganism tested per mg of sample.

In one embodiment, the Archaea microorganism is of the Methanobacteriales order, the Methanomicrobiales order or the Methanosarcinales order. In a particular embodiment of the method of the present invention, the Archaea microorganism is of the Methanobacteriales order. In another embodiment, the Archaea microorganism is of the Methanobacteriacaea family, the Methanosarcinacaea family or the Methanosaetaceae family, preferably the Methanobacteriacaea family. In another embodiment the Archaea microorganism is of the *Methanothermobacter* genus, the *Methanobacterium* genus, the *Methanosphaera* genus or the *Methanobacter* genus. In a further preferred embodiment of the present invention, the Archaea microorganism is of the *Methanobrevibacter* genus.

The *in vitro* method of the invention involves a step (b) comparing the level of Archaea microorganism measured in (a) with a reference level of Archaea microorganism in at least one control sample, wherein an elevated level is indicative of colorectal cancer in said subject. The reference level of Archaea microorganism associated with a control sample or samples refers to the quantity of an Archaea methanogen or Archaea methanogens present in said control, *i.e.* quantification of microbial load in said control sample. Said quantity is determined using the same or analogous methods to that deployed in step (a) of the method from said control sample. As such, the reference level of Archaea microorganism associated with a control sample or samples may be measured and based on the level of Archaea microorganism from an individual control sample or based on the average of multiple control samples. In one embodiment, the reference level(s) of Archaea microorganism in the control sample(s) is measured by real time quantitative polymerase chain reaction. The real time quantitative polymerase chain reaction quantifies the microbial makeup of the control sample based on the number of copies of genes specific to each microorganism tested per mg of sample.

In a particular embodiment of the method disclosed in the present invention, each reference level of Archaea microorganism in a control sample is obtained from a biological sample of a subject not having colorectal cancer, *i.e.* a healthy subject. In the method of the present invention multiple reference levels of an Archaea microorganism from multiple control samples and/or multiple healthy subjects may be compared as a population with the level of Archaea microorganism measured in step (a) of the method. Alternatively, a reference level of Archaea microorganism in a control sample is obtained from one or more subjects having colorectal cancer. Multiple reference levels of Archaea microorganism may be obtained, each reference level being particular to the control population of subjects from which it is obtained. Thus distinct reference levels of Archaea microorganism may be determined in control populations which are classified not only in terms of healthy subjects or subjets having colorectal cancer, but also in terms of factors such as sex, age, ethnic group or clinical condition,

Comparing the level of Archaea microorganism measured in (a) with a reference level of Archaea microorganism in a control sample, means evaluating whether there is a difference between said level and said reference level. Said difference is a numerical difference, preferably a statistically significant difference. When said difference is such that said level is elevated over said reference level, this is indicative of colorectal cancer in said subject, *i.e.* that the subject may be suffering from or susceptible to contracting colorectal cancer.

In the method of the invention when the levels of methanobacteriales are ≥ 3.65 log n° gene copies/mg in a fecal sample obtained from a subject, this is indicative of colorectal cancer in said subject. Moreover, in one embodiment of the method of the invention, when the level of methanobacteriales is ≥ 3.65 log n° gene copies/mg fecal sample obtained from the subject, this is indicative that said subject is suffering from colorectal cancer. In a preferred embodiment of the method of the present invention, suffering from colorectal cancer is indicated by a level of methanobacteriales ≥ 4.00 log n° gene copies/mg fecal sample obtained from the subject, more preferably by a level of methanobacteriales ≥ 4.20 log n° gene copies/mg fecal sample obtained from the subject. In other words, when the level of methanobacteriales is indicative of colorectal cancer in said subject, said subject should seek medical treatment for colorectal cancer or at the very least confirm said diagnosis/prognosis through further tests.

Alternatively, the reference level of Archaea microorganism in a control sample is based on *(i.e.* corresponds with) the specificity of the method of the invention. Specificity is the ability of the method to correctly identify those individuals who do not have colorectal cancer from a population of healthy subjects. For example, the method of the invention is preferably assigned a specificity of 75%, which means that 75 out of 100 patients who do not have colorectal cancer are correctly assigned as being healthy according to said method. A specificity of 75% corresponds with a level of Archaea microorganism of 3.63 log n° gene copies/mg fecal sample. More preferably, the method of the invention has a specificity of 90%, even more preferably 95%, furthermore preferably 99%, yet more preferably 99.9%.

Alternatively, the reference level of Archaea microorganism in a control sample is based on (*i.e.* corresponds with) the sensitivity of the method of the invention. Sensitivity is the ability of the method to correctly identify those who have the disease. For example, the method of the invention is preferably assigned a sensitivity of 75%, which means that 75 out of 100 patients who have colorectal cancer are correctly diagnosed with this disease according to said method. A sensitivity of 75% corresponds with a level of Archaea microorganism of 4.49 log n° gene copies/mg fecal sample. More preferably, the method of the invention has a sensitivity of 90%, even more preferably 95%, furthermore preferably 99%, yet more preferably 99.9%.

In one embodiment of the invention, the level and reference level of Archaea microorganism (in the biological sample and control sample, respectively) is measured using primers of any of SEQ ID NO: 40 to SEQ ID NO: 51. Preferably, primers of any of SEQ ID NO: 40, SEQ ID NO: 41 or SEQ ID NO: 42 are used. In another embodiment of the invention, the level of Archaea microorganism in the biological sample and control sample is measured using primers of either of SEQ ID NO: 52 or SEQ ID NO: 53.

The present invention also relates to an *in vitro* method for diagnosis and/or prognosis of colorectal cancer in a subject, using a biological sample obtained from said subject, which comprises:
(c) measuring the level of *Akkermansia muciniphila* in the biological sample, and
(d) comparing the level of of *Akkermansia muciniphila* measured in (c) with a reference level of *Akkermansia muciniphila* in at least one control sample, wherein a reduced level of *Akkermansia muciniphila* is indicative of colorectal cancer in said subject and/or greater severity of colorectal cancer. Measuring a level of *Akkermansia muciniphila* in the biological sample involves determining the quantity of an *Akkermansia muciniphila* present in said sample, using for example, a real time quantitative polymerase chain reaction (qPCR) or an immunoassay. In a preferred embodiment of the invention, the level of *Akkermansia muciniphila* is measured by real time quantitative polymerase chain reaction.

The aforementioned *in vitro* method of the invention involves a step (d) of comparing the level of of *Akkermansia muciniphila* measured in (c) with a reference level of *Akkermansia muciniphila* in at least one control sample, wherein a reduced level of *Akkermansia muciniphila* is indicative of colorectal cancer in said subject and/or greater severity of colorectal cancer. The reference level of *Akkermansia muciniphila* associated with a control sample or samples refers to the quantity of *Akkermansia muciniphila* present in said control and is determined using the same or analogous methods to that deployed in step (c). As such, in one embodiment, the reference level(s) of *Akkermansia muciniphila* in the control sample(s) is/are measured by real time quantitative polymerase chain reaction.

In a particular embodiment of the method disclosed in the present invention, each reference level of *Akkermansia muciniphila* in a control sample is obtained from a biological sample of a subject not having colorectal cancer, *i.e.* a healthy subject. In the method of the present invention multiple reference levels of an *Akkermansia muciniphila* from multiple control samples and/or multiple healthy subjects may be compared with the level of *Akkermansia muciniphila* measured in step (a) of the method.

Comparing the level of *Akkermansia muciniphila* measured in (a) with a reference level of *Akkermansia muciniphila* in a control sample, means calculating whether there is a difference between said level and said reference level. Said difference is a numerical difference, preferably a statistically significant difference. When said difference is such that said level is below said reference level, this is indicative of colorectal cancer in said subject.

In the *in vitro* method of the invention, a level of *Akkermansia muciniphila* ≤ 4.0 log n° gene copies/mg fecal sample obtained from the subject is indicative of colorectal cancer in said subject and/or greater severity of colorectal cancer. In a preferred embodiment of the method, suffering from colorectal cancer is indicated by a level of *Akkermansia muciniphila* ≤ 3.9 log n° gene copies/mg fecal sample obtained from the subject, more preferably by a level of *Akkermansia muciniphila ≤* 3.8 log n° gene copies/mg fecal sample obtained from the subject. In other words, when the level of *Akkermansia muciniphila* is indicative of colorectal cancer in said subject, said subject should seek medical treatment for colorectal cancer or at the very least confirm said diagnosis/prognosis through further tests.

Alternatively, the reference level of *Akkermansia muciniphila* in a control sample is based on the specificity of the method of the invention. The method of the invention is preferably assigned a specificity of 60%, which means that 60 out of 100 patients who do not have colorectal cancer are correctly assigned as being healthy according to said method. A specificity of 60% corresponds with a level of *Akkermansia muciniphila* of more than 4.0 log n° gene copies/mg fecal sample. More preferably, the method of the invention has a specificity of 65%, even more preferably 70%.

Alternatively, the reference level of *Akkermansia muciniphila* in a control sample is based on the sensitivity of the method of the invention. The method of the invention is preferably assigned a sensitivity of 75%, which means that 75 out of 100 patients who have colorectal cancer are correctly diagnosed with this disease according to said method. A sensitivity of 75% corresponds with a level of *Akkermansia muciniphila* of 3.85 log n° gene copies/mg fecal sample. More preferably, the method of the invention has a sensitivity of 80%, even more preferably 90%, furthermore preferably 99%.

In another preferred embodiment of the method of the invention, the biological sample is characterised in that it is a fecal sample. More preferably, the fecal sample is a fecal sample obtained from a human subject. Even more preferably said human subject is a subject who shows symptoms of colorectal cancer or is experiencing weight loss or changes in bowel habits. Such symptoms may include rectal bleeding, anemia, worsening constipation, decrease in stool calibre, loss of appetite, loss of weight, and nausea or vomiting. Yet more preferably, said human subject is over 50 years of age, furthermore preferably over 65 years of age.

In another aspect, the present invention refers to a kit for *in vitro* detection of colorectal cancer in a biological sample obtained from a subject which comprises:
a) a means for measuring a level of Archaea microorganism and/or a level of *Akkermansia muciniphila* in said biological sample, wherein said Archaea microorganism is a methanogen; and
b) instructions for comparing the level of Archaea microorganism and/or the level of *Akkermansia muciniphila* in said biological sample with a reference level of Archaea microorganism and/or a reference level of *Akkermansia muciniphila* in at least one control sample, wherein an elevated level of Archaea microorganism in said biological sample is indicative of colorectal cancer in said subject and wherein a reduced level of *Akkermansia muciniphila* is indicative of colorectal cancer in said subject and/or greater severity of colorectal cancer in said subject.

Thus, in one embodiment the invention is a kit for *in vitro* detection of colorectal cancer in a biological sample obtained from a subject which comprises:
a) a means for measuring a level of Archaea microorganism in said biological sample, wherein said Archaea microorganism is a methanogen; and
b) instructions for comparing the level of Archaea microorganism in said biological sample with a reference level of Archaea microorganism in at least one control sample,
wherein an elevated level of Archaea microorganism in said biological sample is indicative of colorectal cancer in said subject.

In another embodiment the invention is a kit for *in vitro* detection of colorectal cancer in a biological sample obtained from a subject which comprises:
a) a means for measuring a level of *Akkermansia muciniphila* in said biological sample; and
b) instructions for comparing the level of *Akkermansia muciniphila* in said biological sample with a reference level of *Akkermansia muciniphila* in at least one control sample,
wherein a reduced level of *Akkermansia muciniphila* is indicative of colorectal cancer in said subject and/or greater severity of colorectal cancer in said subject.

In a preferred embodiment of the kit of the invention the means for measuring the level of Archaea microorganism comprises a microarray or a gene chip which comprises commercially available nucleic acid probes, said nucleic acid probes comprising sequences that specifically hybridize to a nucleotide sequence of the Archaea microorganism. In a further preferred embodiment, the nucleic acid probes are selected from any of SEQ ID NO: 40 to SEQ ID NO: 51. In another embodiment the probes are primers of any of SEQ ID NO: 40, SEQ ID NO: 41 or SEQ ID NO: 42. In a yet further preferred embodiment of the invention, said nucleic acid probes are selected from either of SEQ ID NO: 52 or SEQ ID NO: 53.

Similarly, in a preferred embodiment the means for measuring the level of *Akkermansia muciniphila* comprises a microarray or a gene chip which comprises commercially available nucleic acid probes, said nucleic acid probes comprising sequences that specifically hybridize to a nucleotide sequence of *Akkermansia muciniphila.* In a further preferred embodiment, the nucleic acid probes are selected from any of SEQ ID NO: 30 or SEQ ID NO: 31.

In another aspect, the present invention also refers to the use of the method of the present invention or the kit of the present invention in the *in vitro* diagnosis and/or prognosis of colorectal cancer.

### EXAMPLES

The following examples illustrate the invention and should not be considered as limiting, but rather illustrative of the invention.

### Example 1. Subject and Sample collection.

Subjects were selected randomly from the Gastroenterology Unit of the Hospital Universitario Central Asturias (HUCA) in Oviedo, Asturias (Spain). Written informed consent was obtained from the participants and the study protocol was approved by the Principado de Asturias Clinical Research Ethical Committee, Spain. All cases were adults (over 18 years old) who underwent ambulatory colonoscopy for any indication, and in whom neoplastic polyps or colorectal cancer were detected. Asymptomatic patients (over 18 years) included in a program of screening for familial first-degree colorectal cancer as controls were used; and patients with hereditary syndromes (Familial Adenomatous Polyposis, Hereditary Non Polypoid Colorectal Cancer and Positive Familial History of CRC) were excluded.

For this study, stool samples and colorectal biopsies from patients were analyzed. Fecal samples were obtained at least one week before to colonoscopy which were immediately frozen and stored at -80° C, until its microbiological analysis. Total colonoscopy was performed under i.v. sedation in both patients and control participants and they signed a detailed informed consent before its performance. The method used for bowel cleaning for all the participants, was as follows: a) Low-fiber diet for 72 hours, before the colonoscopy. b) Full liquid diet, during the day before the colonoscopy. c) Ingestion of two tablets of bisacodyl-5mg, the day before the colonoscopy around 7 p.m. d) Drinking of 1 l. of Polyethylene Glycol + Ascorbic acid (MoviprepTM) at 8 p.m. on the day before and repeated at the same dose and quantity at 6 a.m. the day of the colonoscopy. The procedure was done in the morning or afternoon, between 9-14 hours.

Biopsy samples were taken from the normal mucosa (rectum) in controls, and from the lesions (polyps and cancer) in patients, for histopathological analysis (Table 1); moreover, two lesion samples were taken for microbiological studies, that were immediately frozen and stored at -80° C, until their analysis.

A total of 28 subjects were included in the study. From those, 7 corresponded to the CRC group, 11 to the tubular adenoma (polyp) group and 10 healthy individuals to the control group. There were 7 males in the CRC group, and 10 males and 1 female in the polyp group. In the healthy group, 6 males and 4 females were included. The mean (±SD) age of the subjects was 71.1 ± 10.1 years for the CRC group and 63.3 ± 13.1 years for the polyp group, whereas the age of the healthy group was 52.6 ± 15.2 years. One biopsy and one fecal sample were collected from each patient and each healthy subject, with some exceptions. Finally, it was collected 4 complete sets of samples for the healthy group, 6 for the polyp group and 7 for the adenocarcinoma group. Patient characteristics and details of surgical treatment are shown in **Table 1.**

**Table 1.** Subjects and Samples included in the study. A sample of normal mucosa and of either polyp or tumor was obtained from patients. TNM system describes the size and/or extent (reach) of the primary tumor (T), the absence/presence of methastasis in close lymph nodes (N) and the presence/absence of distal metastasis (M).

| **N°** | **AGE** | **GENDER** | **LOCATION** | **PATHOLOGY** | **UNDERWENT SURGERY** | **TNM** | **BIOPSY** | **FECES** |
|---|---|---|---|---|---|---|---|---|
| **CONTROL** | | | | | | | | |
| 7 | 55 | F | Rectum | Normal | No | - | No | Yes |
| 9 | 18 | M | Rectum | Normal | No | - | No | Yes |
| 10 | 56 | M | Rectum | Normal | No | - | No | Yes |
| 12 | 63 | F | Rectum | Normal | No | - | No | Yes |
| 14 | 61 | F | Rectum | Normal | No | - | Yes | Yes |
| 16 | 69 | M | Rectum | Normal | No | - | Yes | Yes |
| 20 | 62 | M | Rectum | Normal | No | - | Yes | Yes |
| 23 | 42 | M | Rectum | Normal | No | - | Yes | No |
| 26 | 60 | F | Rectum | Normal | No | - | Yes | Yes |
| 31 | 40 | M | Rectum | Normal | No | - | No | Yes |

| **POLYP** | | | | | | | | |
|---|---|---|---|---|---|---|---|---|
| 3 | 61 | M | Transverse | Tubular Adenoma | No | - | Yes | No |
| 8 | 41 | M | Sigmoid | Tubular Adenoma | No | - | No | Yes |
| 13 | 85 | F | Cecum | Tubular Adenoma | No | - | Yes | Yes |
| 17 | 63 | M | Rectum | Villous Adenoma | No | - | Yes | Yes |
| 18 | 78 | M | Cecum | Tubular Adenoma | No | - | Yes | Yes |
| 19 | 49 | M | Descending | Tubular Adenoma | No | - | Yes | Yes |
| 24 | 80 | M | Rectum (several polyps) | Tubular Adenoma | No | - | Yes | No |
| 25 | 59 | M | Sigmoid | Tubular Adenoma | No | - | Yes | Yes |
| 27 | 60 | M | Sigmoid | Tubular Adenoma | No | - | Yes | Yes |
| 28 | 61 | M | Cecum | Tubular Adenoma | No | - | Yes | No |
| 32 | 59 | M | Cecum | Tubular Adenoma | No | - | No | Yes |

| **TUMOR** | | | | | | | | |
|---|---|---|---|---|---|---|---|---|
| 6 | 81 | M | Sigmoid | Adenocarcinoma | Yes | T2N1M0 | Yes | Yes |
| 15 | 72 | M | Sigmoid | Adenocarcinoma | Yes | T3N1M0 | Yes | Yes |
| 21 | 56 | M | Transverse | Adenocarcinoma | Yes | T3N0M0 | Yes | Yes |
| 22 | 65 | M | Rectum | Adenocarcinoma | Yes | T2N0M0 | Yes | Yes |
| 29 | 79 | M | Rectum | Adenocarcinoma | Yes | T3N0M0 | Yes | Yes |
| 30 | 82 | M | Descending | Adenocarcinoma | Yes | T2N0M0 | Yes | Yes |
| 33 | 63 | M | Sigmoid | Adenocarcinoma | Yes | T2N0M0 | Yes | Yes |

### Example 2. DNA extraction.

Fecal DNA was extracted using the QIAamp DNA stool mini kit (Qiagen). Briefly, 220-250mg portions of frozen faeces were taken and resuspended in lysis buffer and disgregated by bead beating with glass beads (0.17 mm diameter) in order to release bacterial cells from the rest of organic matter in the feaces. After this previous step the samples were heated at 95°C for 5 min to improve bacterial lysis and processed according to the manufacturer's protocol. DNA was collected in 200 µL of MiliQ water for further studies.

Bacterial DNA extraction from 5 to 15 mg biopsies was performed with NucleoSpin Tissue extraction kit (Macherey-Nagel, Germany) in order to minimize the ratio of human DNA from intestinal epithelial cells. DNA was isolated according to the manufacturer's instructions with a previous glass bead beating, and finally eluted in 60µL of MiliQ water for further studies. All samples were evaluated in 0.7% agarose gels. DNA concentration and integrity of the extracted DNA was measured by calculating A260/280 ratios in a Quawell Spectrophotometer (Thermo Fisher Scientific).

### Example 3. 16S rRNA gene pyrosequencing.

The first 500 bp of the 16S rRNA genes were amplified with the universal eubacterial primers 27F and 533R [Titanium-LibL-A-27F-01 to Titanium-LibL-A-27F-20 (SEQ ID No. 1 to SEQ ID No. 20) and Titanium-LibL-B-533R (SEQ ID No. 21:)] using the high-fidelity AB-Gene DNA polymerase (Thermo Scientific) with an annealing temperature of 52°C and 20 cycles to minimize PCR biases, following Cabrera-Rubio et al. (Cabrera-Rubio R, et al. J Clin Microbiol; 2012; 50:3562-3568 Microbiome diversity in the bronchial tracts of patients with chronic obstructive pulmonary disease). The universal primers 454 for amplifying the bacterial gene 16S in one direction (forward) were modified to contain the pyrosequencing adaptors A and B of the chemical Titanium corresponding to the kits of the type "shot-gun" Lib-L. Barcodes were different in at least 3 nucleotides from each other to avoid mistakes in sample assignments. In the reverse direction a Titanium primer with the pyrosequencing adaptor B was used for all samples. The sequences of universal eubacterial primers 27F after suffering the modifications mentioned above are the following, wherein the adaptor and the 8 bp corresponding to the bar code are underlined. The other two are those of the separator (linker) and the rest correspond to the forward primer 8-27F, degenerating:and an 8bp "barcode" specific to each sample.
SEQ ID No. 1 Titanium-LibL-A-27F-01
   CCATCTCATCCCTGCGTGTCTCCGACTCAGAACCAACCTCAGAGTTTGATCMTGGCTCAG
SEQ ID No. 2 Titanium-LibL-A-27F-02
   CCATCTCATCCCTGCGTGTCTCCGACTCAGAACCGGAATCAGAGTTTGATCMTGGCTCAG
SEQ ID No. 3 Titanium-LibL-A-27F-03
   CCATCTCATCCCTGCGTGTCTCCGACTCAGAACCTTCCTCAGAGTTTGATCMTGGCTCAG
SEQ ID No. 4 Titanium-LibL-A-27F-04
   CCATCTCATCCCTGCGTGTCTCCGACTCAGAAGCCGAATCAGAGTTTGATCMTGGCTCAG
SEQ ID No. 5 Titanium-LibL-A-27F-05
   CCATCTCATCCCTGCGTGTCTCCGACTCAGAAGCGGATTCAGAGTTTGATCMTGGCTCAG
SEQ ID No. 6 Titanium-LibL-A-27F-06
   CCATCTCATCCCTGCGTGTCTCCGACTCAGAAGGTTCCTCAGAGTTTGATCMTGGCTCAG
SEQ ID No. 7 Titanium-LibL-A-27F-07
   CCATCTCATCCCTGCGTGTCTCCGACTCAGAATACGCCTCAGAGTTTGATCMTGGCTCAG
SEQ ID No. 8 Titanium-LibL-A-27F-08
   CCATCTCATCCCTGCGTGTCTCCGACTCAGACACCACATCAGAGTTTGATCMTGGCTCAG
SEQ ID No. 9 Titanium-LibL-A-27F-09
   CCATCTCATCCCTGCGTGTCTCCGACTCAGACATCTCTTCAGAGTTTGATCMTGGCTCAG
SEQ ID No. 10 Titanium-LibL-A-27F-10
   CCATCTCATCCCTGCGTGTCTCCGACTCAGACAGACTCTCAGAGTTTGATCMTGGCTCAG
SEQ ID No. 11 Titanium-LibL-A-27F-11
   CCATCTCATCCCTGCGTGTCTCCGACTCAGACCAACCATCAGAGTTTGATCMTGGCTCAG
SEQ ID No. 12 Titanium-LibL-A-27F-12
   CCATCTCATCCCTGCGTGTCTCCGACTCAGACCAAGCTTCAGAGTTTGATCMTGGCTCAG
SEQ ID No. 13 Titanium-LibL-A-27F-13
   CCATCTCATCCCTGCGTGTCTCCGACTCAGACCTAGGTTCAGAGTTTGATCMTGGCTCAG
SEQ ID No. 14 Titanium-LibL-A-27F-14
   CCATCTCATCCCTGCGTGTCTCCGACTCAGACCTTCCATCAGAGTTTGATCMTGGCTCAG
SEQ ID No. 15 Titanium-LibL-A-27F-15
   CCATCTCATCCCTGCGTGTCTCCGACTCAGACGAAGCATCAGAGTTTGATCMTGGCTCAG
SEQ ID No. 16 Titanium-LibL-A-27F-16
   CCATCTCATCCCTGCGTGTCTCCGACTCAGACTCGACATCAGAGTTTGATCMTGGCTCAG
SEQ ID No. 17 Titanium-LibL-A-27F-17
   CCATCTCATCCCTGCGTGTCTCCGACTCAGACTCGAGTTCAGAGTTTGATCMTGGCTCAG
SEQ ID No. 18 Titanium-LibL-A-27F-18
   CCATCTCATCCCTGCGTGTCTCCGACTCAGACTGACACTCAGAGTTTGATCMTGGCTCAG
SEQ ID No. 19 Titanium-LibL-A-27F-19
   CCATCTCATCCCTGCGTGTCTCCGACTCAGAGACGACATCAGAGTTTGATCMTGGCTCAG
SEQ ID No. 20 Titanium-LibL-A-27F-20
   CCATCTCATCCCTGCGTGTCTCCGACTCAGAGAGTCTCTCAGAGTTTGATCMTGGCTCAG

Similarly, the sequence of universal eubacterial primer 533R after suffering the modifications mentioned above is the following, wherein the adaptor is underlined.
SEQ ID No. 21 Titanium-LibL-B-533R
   CCTATCCCCTGTGTGCCTTGGCAGTCTCAGGCTTACCGCGGCKGCTGGCACG

Two PCRs were used per sample, pooling their PCR products before purification, which was performed using an Nucleofast PCR purification kit (Macherey-Nagel) and further cleaned by AMPure XP beads (Roche, Basel, Switzerland) before pyrosequencing. The final DNA concentration per sample was measured by picogreen fluorescence in a Modulus 9200 fluorimeter from Turner Biosystems, and samples were mixed in equimolar amounts in groups of 20. PCR products were pyrosequenced from the forward primer end only in 1/8th of pyrosequencing plates using a GS-FLX sequencer with Titanium chemistry (Roche) at the Center for Public Health Research (CSISP-FISABIO) in Valencia, Spain.

### Example 4. Sequence Analysis.

Reads with an average quality value lower than 20 and/or with more than 4 ambiguities in homopolymeric regions in the first 360 flows were excluded from the analysis. Only reads longer than 200 bp were considered, and chimeric sequences were filtered out using Mothur (Schloss PD, et al. Appl Environ Microbiol. 2009; 75(23):7537-41). The ends of reads were found to have an increased rate of ambiguous basecalls and sequences were therefore end-trimmed in 10-bp windows of mean quality values <20 using Prinseq. Sequences were assigned to each sample by the 8-bp barcode and passed through the Ribosomal Database Project classifier (Cole JR, et al. Nucleic Acids Res. 2009 Jan;37(Database issue):D141-5), where each read was assigned a phylum, class, family and genus, as long as the taxonomic assignment was unambiguous within an 80% confidence threshold. To estimate total diversity, sequences were clustered at 97% nucleotide identity over 90% sequence alignment length using the RDP pyrosequencing pipeline, and rarefaction curves obtained with the program Analytic Rarefaction 1.3. For this analysis, sequences over 97% identical were considered to correspond to the same Operational Taxonomic Unit (OTUs), representing a group of reads which likely belong to the same species (Yarza P, et al. Syst Appl Microbiol. 2008; 31: 241-250). Principal Coordinates Analyses (PCoA) were performed with UNIFRAC (Lozupone C, et al. Appl Environ Microbiol. 2005, 71 (12):8228-8235) using clustering at 97% sequence identity. The Unifrac analysis compares the 16S-estimated diversity with a phylogenetic approach that takes into account both taxonomically assigned and unassigned reads.

### Example 5. Archaea analysis using real-time quantitative PCR.

Quantitative real-time PCR was performed using ABI Prism 7900HT (Applied Biosystems) with four different primers and probe sets **(Table 2):** two hidrogenotrophic orders (Methanomicrobiales (MMB) and Methanobacteriales (MBT)) and two acetoclastic families (Methanosaetaceae (Mst) and Methanosarcinaceae (Msc)). Each reaction mixture was carried out in a total volume of 10 µl containing genomic DNA, 1X Taqman universal PCR Master Mix (Applied Biosystems), 0.9 µM of forward and reverse primer and 0.2 µM of the probe. The Taqman Probes were labeled with the fluorescent dyes FAM (reporter) and TAMRA (quencher). The presence of Methanobrevibacter genus (MET) and *Methanobrevibacter smithii* (Mnif) by specific-qPCR in a LightCycler® 480 Real-Time PCR System (Roche) using SYBR® Green PCR Master Mix (Roche) was also analysed. Each reaction mixture of 10µl was composed of 0.5 µl of each of the specific primers at a concentration of 0.25 µM, and 1 µl of template DNA.

All amplifications were performed in triplicate and a corresponding standard curve was included. Triplicate results of the qPCR were averaged and the standard deviation calculated. The thermal cycling protocol was as follows: initial denaturation of 10min at 95°C followed by 45°C denaturation cycles at 95°C for 15s, annealing at 50°C (Mst-set, Msc-set) or 54°C (MBT-set) or 55°C (MET and Mnif) for 30s and extension at 72°C for 30s. For MMB primer set the amplification was carried out using a two-step thermal cycling protocol consisting in 45 cycles of 15s at 95°C and 90s at 63°C.

Standard curves were generated using a plasmid containing the full-length 16S rRNA gene sequences from the representative strains of the target methanogenic group as described previously by Yu *et al.* (Yu Y, et al. Biotechnol. Bioeng. 2005; 20;89(6):670-9). Genomic DNA from four archaea (*Methanobacterium formicicum* DSM 1535; *Methanoculleus bourgensis* DSM 3045; *Methanosarcina barkeri* DSM 800 and *Methanosaeta concilii* DSM 2139; *Methanobrevibacter smithii* DSM 2374) were used for standard curves. DNA concentration was measured with the Picogreen double-stranded DNA quantification kit (Invitrogen) and 10-fold serial dilution series (10²-10⁹ copies) was generated for each standard curve and analysed by quantitative PCR in triplicate with its corresponding primer and probe set.

**Table 2. Primers used in the qPCR analysis.**

| **Bacterial groups** | **Name** | **Sequence Number** |
|---|---|---|
| *Bifidobacterium* genus | Forward | SEQ ID No. 22 |
| | Reverse | SEQ ID No. 23 |
| *Blautia coccoides* group | Forward | SEQ ID No. 24 |
| | Reverse | SEQ ID No. 25 |
| *Bacteroides-Prevotella* | Forward | SEQ ID No. 26 |
| | Reverse | SEQ ID No. 27 |
| *Lactobacillus* spp. | Forward | SEQ ID No. 28 |
| | Reverse | SEQ ID No. 29 |
| *Akkermansia muciniphila* | Forward | SEQ ID No. 30 |
| | Reverse | SEQ ID No. 31 |
| Enterobacteriaceae family | Forward | SEQ ID No. 32 |
| | Reverse | SEQ ID No. 33 |
| *Total Bacteria* | Forward | SEQ ID No. 34 |
| | Reverse | SEQ ID No. 35 |
| *Faecalibacterium prausnitzii* | Forward | SEQ ID No. 36 |
| | Reverse | SEQ ID No. 37 |
| *Fusobacterium nucleatum* | Forward | SEQ ID No. 38 |
| | Reverse | SEQ ID No. 39 |

| **Archaea groups** | | |
|---|---|---|
| *Methanobacteriales* | MBT857F | SEQ ID No. 40 |
| | MBT929F-Taqman | SEQ ID No. 41 |
| | MBT1196R | SEQ ID No. 42 |
| *Methanomicrobiales* | MMB282F | SEQ ID No. 43 |
| | MMB749F-Taqman | SEQ ID No. 44 |
| | MMB832R | SEQ ID No. 45 |
| *Methanosarcinaceae* | Msc380F | SEQ ID No. 46 |
| | Msc492F-Taqman | SEQ ID No. 47 |
| | Msc828R | SEQ ID No. 48 |
| *Methanosaetaceae* | Mst702F | SEQ ID No. 49 |
| | Mst753F-Taqman | SEQ ID No. 50 |
| | Mst862R | SEQ ID No. 51 |
| *Methanobrevibacterium* | MET-105f | SEQ ID No. 52 |
| | MET-386r | SEQ ID No. 53 |
| *M. smithii* | Mnif-342f | SEQ ID No. 54 |
| | Mnif-363r | SEQ ID No. 55 |

**Table 3. Values of Methanobacteriales in feces of healthy subjects, subjects with polyps and subjects with tumors**

| **Subject** | **Subject type** | **gene copies/mg sample** | **Log (gene copies/mg sample)** | **Average, Std. Dev.** | **Average, Std. Dev.** |
|---|---|---|---|---|---|
| H7 | Healthy | 4227 | 3.63 | Av. 3.72 S.D. 0.79 | Av. 3.72 S.D. 0.79 |
| H9 | Healthy | 2706 | 3.43 | | |
| H10 | Healthy | 264129 | 5.42 | | |
| H12 | Healthy | 2930 | 3.47 | | |
| H14 | Healthy | 1941 | 3.29 | | |
| H16 | Healthy | 2453 | 3.39 | | |
| H20 | Healthy | 1538 | 3.19 | | |
| H26 | Healthy | 8209 | 3.91 | | |
| H31 | Healthy | 2306 | 3.36 | | |
| H8 | Polyp | 938 | 2.97 | Av. 4.78 S.D. 1.48 | Av. 5.12 S.D: 1.67 |
| H13 | Polyp | 2044 | 3.31 | | |
| H17 | Polyp | 2205 | 3.34 | | |
| H18 | Polyp | 92238 | 4.96 | | |
| H19 | Polyp | 127195 | 5.10 | | |
| H25 | Polyp | 1222757 | 6.09 | | |
| H27 | Polyp | 13134236 | 7.12 | | |
| H32 | Polyp | 3298 | 3.52 | | |
| H6 | Tumor | 1919335 | 6.28 | Av. 5.76 S.D. 1.71 | |
| H15 | Tumor | 4412311 | 6.64 | | |
| H21 | Tumor | 2582935 | 6.41 | | |
| H22 | Tumor | 331159062 | 8.52 | | |
| H29 | Tumor | 19211 | 4.28 | | |
| H30 | Tumor | 49195 | 4.69 | | |
| H33 | Tumor | 2988 | 3.48 | | |

### Example 6. Bacterial analysis using real-time quantitative PCR.

PCR primers were targeted to total bacteria count and to *Bifidobacterium* spp., *Lactobacillus* spp.; *Bacteroides-Prevotella* group; *Enterobacteriaceae* family; *Blautia* coccoides group; *Akkermansia muciniphila; Faecalibacterium prausnitzii & Fusobacterium nucleatum.* The qPCRs were conducted as previously described (Collado M C, et al. J Clin Nutr. 2008; 88: 894-899). The qPCR amplification and detection were performed in a LightCycler® 480 Real-Time PCR System (Roche). Each reaction mixture of 10µl was composed of SYBR® Green PCR Master Mix (Roche), 0.5 µl of each of the specific primers at a concentration of 0.25 µM, and 1 µl of template DNA. The fluorescent products were detected in the last step of each cycle. A melting curve analysis was made after amplification to distinguish the targeted from the non-targeted PCR product. The bacterial concentration in each sample was calculated comparing the Ct values obtained from standard curves. These were created using serial 10-fold dilution of pure culture-specific DNA fragments corresponding to 10 to 10⁹ gene copies/ml.

### Example 7. qPCR data statistical analysis.

For statistical analysis, SPSS 17.0 software was used. Due to the non-normal distribution of microbial data, they were expressed as medians with interquartile ranges (IQR) and non-parametric tests were performed. Friedman's test was used to compare microbial groups through time (paired samples). Mann-Whitney U test was used for comparisons between two groups. For comparisons of more than two groups, Kruskal-Wallis test was applied, and statistical differences were corrected for a multiple comparison test using the Bonferroni correction. The X-square test was applied to establish differences in bacterial prevalence between the studied groups. A p-value <0.05 was considered statistically significant. Spearman rank test allowed the study of the correlation between variables, and significance was established at a coefficient of 0.5%.

### Example 8. Mucosal and fecal microbiota composition by 16S rRNA gene pyrosequencing.

After sequence length and quality filtering, an average of 3494 reads of the 16S rRNA gene per sample was obtained. Sequences are available in the MG-RAST server with accession numbers 4542409.3 through 4542468.3 under the project name "Colorectal Cancer Microbiota". The 16S rRNA reads were grouped by sample type, and the number of sequence clusters at 97% of sequence identity was used as an estimate of the approximate number of species, given that a 3% of divergence in this gene has been established as the threshold for prokaryotic species boundaries (Yarza P, et al. Syst Appl Microbiol. 2008; 31: 241-250). The number of species-level "Operational Taxonomic Units" (OTUs) was related to the sequencing effort in rarefaction curves, where the level of bacterial diversity can be compared between sample types (**Figure 1****).** Tissue samples were found to have almost half of the diversity observed in fecal samples (**Figure 1A**)**.**

When the different biopsies were analyzed, tumor samples displayed the highest level of diversity, having an estimated number of species 75% higher than tissues from healthy sites, which were used as a control (**Figure 1B**)**.** Polyp samples displayed intermediate diversity values. The higher bacterial diversity detected at diseased sites is contrary to the general trend of higher diversity observed in healthy human tissues in inflammatory or infectious diseases compared to diseased sites. This has been interpreted as enrichment at diseased sites of a reduced number of microorganisms that thrive at the specific conditions of the new niche, at the expense of many other microbial species which would be more sensitive to environmental changes.

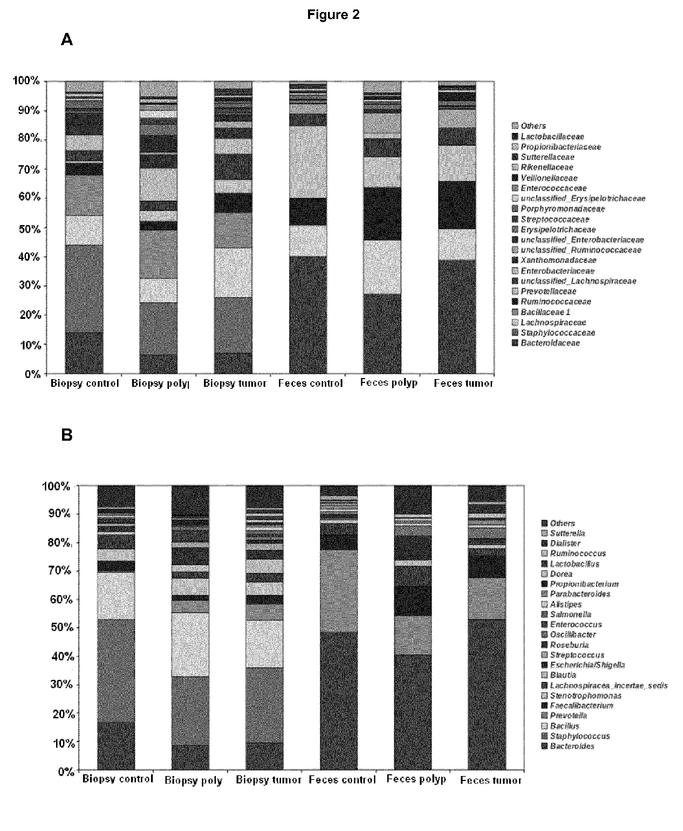

The bacterial composition of different samples, as estimated by assignment of the 16S rRNA reads to the RDP database, shows that feces and biopsies contain very different bacterial communities, with some genera like *Bacillus* or *Staphylococcus* that were absent in fecal material (**Figure 2**)**.** In addition, all three fecal samples (from individuals having polyps or tumors, as well as from healthy controls) showed a similar bacterial composition at the genus taxonomic level. Tissue samples show differences between healthy and affected tissues, with several genera, like *Blautia* and *Prevotella* which were virtually absent in healthy tissues but present in similar proportion in polyps and tumors, and that could therefore be used as potential biomarkers of the disease (**Figure 2**)**,** but in an invasive method of diagnosis/prognosis of the colorectal cancer.

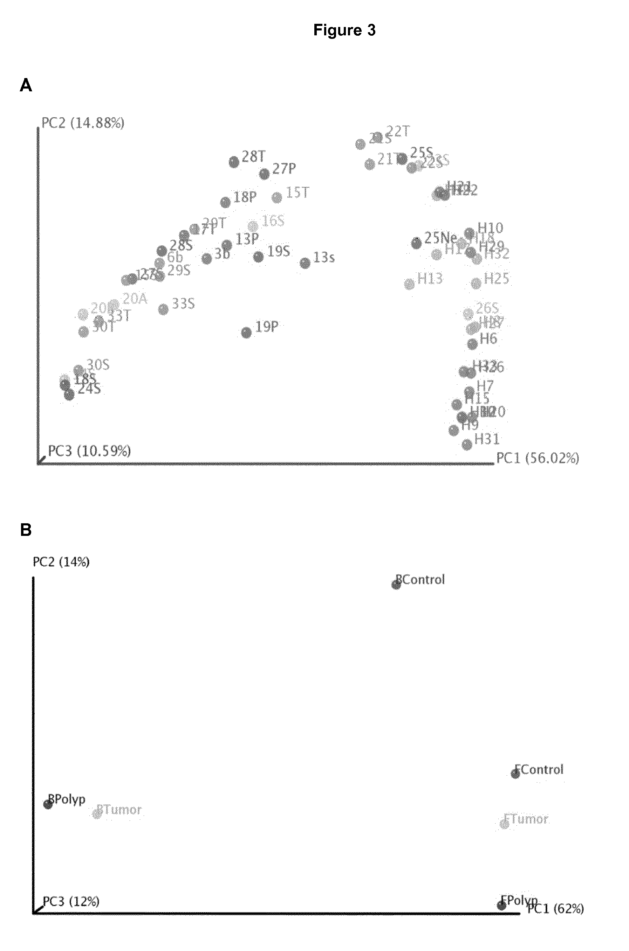

Current sequence length of pyrosequencing reads makes taxonomic assignment at the genus level very reliable, but it is not sufficient for accurate species-level assignment. However, species-level Principal Coordinates Analysis (PCoA) is independent of taxonomic assignment and allows a graphic representation where samples occupy a position in a 3D space depending on their bacterial composition [Lozupone C, et al. Appl Environ Microbiol. 2005, 71(12):8228-8235]. When a PCoA was performed with the samples grouped by sample type, over 90% of the variability in the data could be explained by the first three components of the analysis (**Figure 3**)**.** The principal component of the analysis clearly separated healthy tissue samples from polyps and tumors, supporting the presence of specific bacterial consortia that are associated to affected sites and that can serve as bacterial biomarkers of colorectal cancer progression, but as it has been mentioned above, in an invasive method of prognosis/diagnosis of colorectal cancer.

### Example 9. Mucosal and fecal microbiota composition (Archaea and Bacteria) by qPCR.

The inventors have observed differences on microbiota composition between patients suffering CRC (n=15) compared to those who had normal colon (n=9). From the 4 Archaea groups analysed, Methanobacteriales was the unique group detected in the samples analysed (**Table 4**) and *Methanobrevibacterium* was included as major representative genus in human colon.

**Table 4. Microbiota composition of fecal samples grouped by type of case (polyp and tumor) detected by qPCR.**

| | **Log n° gene copies /mg** | | | | | | **Mann-Whitney U-test** | **KruskalWallis Test** |
|---|---|---|---|---|---|---|---|---|
| **Bacteria** | **Pr** | **Control (n=9)** | **Case (n=15)** | | | | | |
| | | | **Pr** | **Polyp (n=8)** | **Pr** | **Tumor (n=7)** | **P-value1** | **P-value2** |
| Total Bacteria | 9 | 7.88 (7.72-8.28) | 8 | 8.00 (7.59-8.29) | 7 | 8.20 (7.95-8.50) | 0.355 | 0.563 |
| *Bifidobacterium* group | 9 | 6.09 (5.42-6.32) | 8 | 5.67 (4.74-6.15) | 7 | 5.60 (5.16-6.10) | 0.986 | 0.189 |
| *Blautia. coccoides* group | 9 | 7.27 (7.08-7.46) | 8 | 7.31 (6.84-7.58) | 7 | 7.25 (7.18-7.63) | 0.990 | 0.926 |
| Enterobacteriaceae family | 9 | 4.41 (3.96-5.52) | 8 | 4.51 (4.33-5.77) | 7 | 5.25 (4.65-5.97) | 0.298 | 0.397 |
| *Lactobacillus* group | 9 | 2.93 (2.52-3.23) | 8 | 3.06 (2.64-4.50) | 7 | 3.59 (2.68-5.83) | 0.487 | 0.233 |
| *Bacteroides-Prevotella group* | 9 | 7.40 (6.98-7.74) | 8 | 7.33 (6.67-7.47) | 7 | 7.36 (6.89-7.83) | 0.487 | 0.789 |
| *Faecalibacterium prausnitzii* | 9 | 5.15 (4.64-5.34) | 8 | 5.25 (4.96-5.50) | 7 | 5.19 (5.01-5.65) | 0.954 | 0.687 |
| *Akkermansia muciniphila* | 5 | 4.90 (3.47-4.85) | 4 | 2.97 (2.30-4.39) | 5 | 3.71 (3.21-4.32) | 0.327 | 0.198 |
| *Fusobacterium nucleatum* | 2 | 4.16 (3.47-4.85) | 3 | 4.26 (4.14-4.52) | 6 | 4.70 (3.85-5.15) | 0.439 | 0.659 |

| **Archae** | | | | | | | | |
|---|---|---|---|---|---|---|---|---|
| *Methanobacteriales* | 9 | 3.43 (3.36-3.63) | 8 | 4.24 (3.34-5.35) | 7 | 6.28 (4.49-6.53) | 0.165 | **0.031** |
| *Methanobrevibacter* | 9 | 3.10 (2.88-3.55) | 8 | 3.75 (2.88-5.30) | 7 | 5.60 (3.84-6.00) | 0.129 | **0.019** |

Data was obtained from positive samples and are shown as median and interquartile range (IQR). Statistical analysis was calculated using the Kruskal-Wallis test. Statistical differences were corrected for a multiple comparison test using the Bonferroni adjustment, and significant differences among groups were considered as having a P-value <0.017 (0.05/3). P-value 1= comparison between tumor and polyp groups. P-value 2= comparison between 3 groups.

Higher levels of *Bifidobacterium* spp. (P-value= 0.069) and lower levels of Methanobacteriales (P-value= 0.040) and *Methanobrevibacterium* (P-value=0.030) were present in fecal samples from healthy subjects compared to the CRC group (polyp and tumor, n=15). Higher presence of *Fusobacterium nucleatum* in feces (P- value= 0.07) was observed in CRC compared to healthy controls. Interestingly, higher levels and higher abundance of Enterobacteriaceae in mucosal samples from CRC groups than in healthy controls (P-value=0.034 and prevalences 7/15, 46.6% vs 0/5, 0% & X2 test P-value=0.05, respectively) were found. *Faecalibacterium* presence was also lower in the CRC group compared to controls (2/15, 13.3% vs 2/5, 40% & X2 test P-value=0.06); however the presence of *Akkermansia muciniphila* was higher in mucosal samples from CRC than in controls (5/15, 33.3% & vs 0/5, 0%, X2 test p-value=0.136). Moreover, a higher presence of *Blautia coccoides* in CRC than in control samples (13/15, 86.7% vs 2/5, 40% & X2 test P-value=0.036) were observed.

When CRC samples are divided in two different disease phases: tubular adenoma (polyp group) and adenocarcinoma (tumor group), it also observed microbiota composition differences in both feces and mucosal samples (**Table 4** and **5**). Lower, although not statistically significant levels of *Bifidobacterium* spp (p-value=0.081) and higher levels of *Lactobacillus* (P-value=0.064) were detected in fecal samples from controls compared to those observed in the tumor group. Significantly higher levels of Enterobacteriaceae were found in mucosal samples from tumor compared to polyp groups (p-value=0.035). Moreover, it was also found higher presence of *Blautia coccoides* in tumor mucosal samples than in polyp biopsies (7/7, 100% vs 6/9, 66.6% & X2 test p-value=0.09).

**Table 5. Microbiota composition of biopsies samples grouped by type of case (polip and tumor) detected by qPCR.**

| | **Log n° gene copies /mg** | | | | | | **Mann-Whitney U-test** | **KruskalWallis Test** |
|---|---|---|---|---|---|---|---|---|
| **Bacteria** | **Pr** | **Control (n=5)** | **Case (n=15)** | | | | | |
| | | | **Pr** | **Polyp (n=8)** | **Pr** | **Tumor (n=7)** | **P-value1** | **P-value2** |
| Total Bacteria | 5 | 5.56 (4.03-6.03) | 8 | 5.09 (4.72-5.52) | 7 | 5.78 (5.03-6.08) | 0.266 | 0.549 |
| *Bifidobacterium* group | 1 | - | 2 | 3.90 (2.95-4.02) | 2 | 3.58 (3.01-4.86) | 0.986 | 0.741 |
| *Blautia. coccoides* group | 2 | 5.61 (5.57-5.66) | 6 | 4.41 (4.31-5.05) | 7 | 4.53 (3.65-5.28) | 0.886 | 0.309 |
| Enterobacteriaceae family | - | - | 3 | - | 4 | 3.80 (3.07-4.35) | 0.035* | 0.034 |
| *Lactobacillus* group | 1 | - | - | - | 3 | 3.71 (3.64-4.16) | - | 0.180 |
| *Bacteroides group* | 2 | 5.25 (5.00-5.51) | 5 | 4.09 (3.89-5.20) | 6 | 4.15 (3.70-5.10) | 0.855 | 0.379 |
| *Faecalibacterium prausnitzii* | 2 | 3.12 (3.07-3.18) | 1 | - | 1 | - | 0.317 | 0.259 |
| *Akkermansia muciniphila* | - | | 2 | 2.10 (2.00-2.20) | 3 | 2.20 (2.07-2.40) | 0.564 | 0.564 |
| *Fusobacterium nucleatum* | - | - | 2 | 5.25 (4.62-5.75) | 2 | 4.55 (4.40-5.80) | - | 0.439 |

| **Arhaea** | | | | | | | | |
|---|---|---|---|---|---|---|---|---|
| *Methanobacteriales* | 5 | 4.38 (4.02-4.39) | 8 | 4.27 (4.11-4.52) | 7 | 4.87 (4.25-5.02) | 0.247 | 0.340 |
| *Methanobrevibacter* | 3 | 3.50 (2.88-3.60) | 5 | 3.90 (3.25-4.08) | 5 | 3.72 (3.27-4.60) | 0.754 | 0.590 |
| *M. smithii* | 3 | 3.34 (3.30-3.40) | 5 | 3.82 (3.22-4.13) | 5 | 3.66 (3.34-4.26) | 0.808 | 0.872 |

Data was obtained from positive samples and are shown as median and interquartile range (IQR). Statistical analysis was calculated using the Kruskal-Wallis test. Statistical differences were corrected for a multiple comparison test using the Bonferroni adjustment, and significant differences among groups were considered as having a P-value <0.017 (0.05/3).P-value 1 = comparison between tumor and polyp groups. P-value 2= comparison between 3 groups.

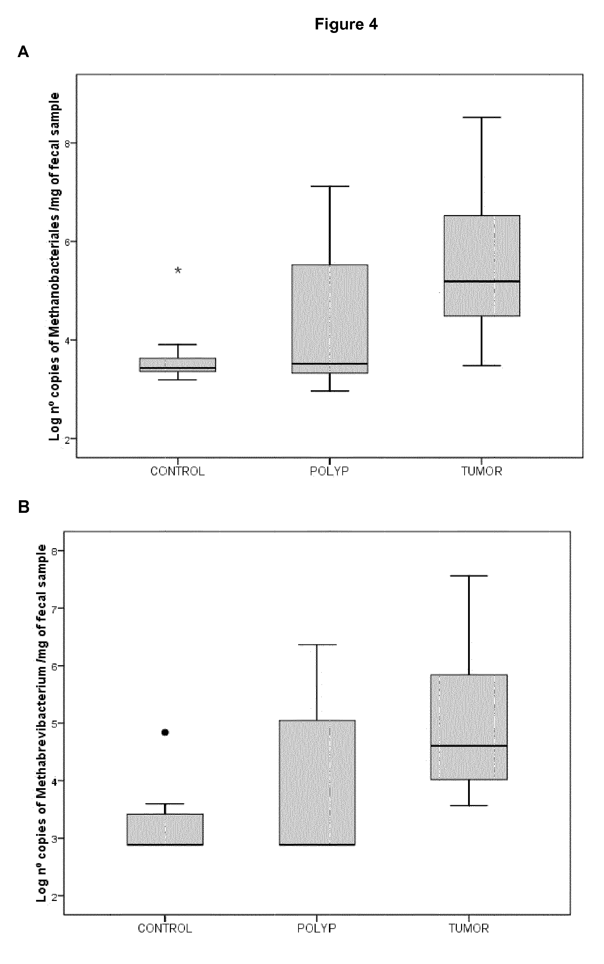

Furthermore, significantly higher levels of fecal methanobacteriales were observed comparing control group and tumor (P =0.0033) (**Figure 4A**)**,** but no differences between controls and adenoma (colonic polyp) (*P*=0.48). Additionally no significant differences between tumor and polyp samples were found (*P*=0.189). *Methanobrevibacterium* was also significantly higher in fecal samples from tumor group than in controls (*P*=0.003) (**Figure 4B**); however no differences were found in *M. smithii* (*P*=0.136). We observed a correlation between the CRC process development and fecal levels of methanobacteriales (R=0.537, *P* =0.007) as well as with the levels of *Methanobrevibacterium* genus (R=0.574, *P*=0.03). The highest levels of Methanobacteriales were observed in fecal samples from adenocarcinoma patients with tumor located in descending colon and in rectum (**Table 6**).

**Table 6. Microbiota composition of fecal samples in control and patients with tumor detected by qPCR.**

| | **Log n° gene copies /mg** | | | | **Kruskal-Wallis Test** |
|---|---|---|---|---|---|
| **FECES (n=16)** | **Pr** | **Control (n=9)** | **Pr** | **Patients with tumor (n=7)** | **P-value** |
| Methanobacteriales | 9 | 3.43 (3.36-3.63) | 7 | 6.28 (4.49-6.53)* | **0.0314** |

| | | | | | |
|---|---|---|---|---|---|
| Data was obtained from positive samples and are shown as median and interquartile range (IQR). Statistical analysis was calculated using the Kruskal-Wallis test. * P-value = 0.0033. | | | | | |

**Table 7. Quantification of Methanobacteriales present in samples of feces of patients (tumor and polyps) and control subjects (healthy).**

| | **Log n° gene copies / mg** | | | | **Mann-Whitney Test** |
|---|---|---|---|---|---|
| **FECES (n=24)** | **Pr** | **Control (n=9)** | **Pr** | **Patients (n=15)** | **P-value** |
| Methanobacteriales | 9 | 3.43 (3.36-3.63) | 15 | 4.96 (3.50-6.35) | 0.0426* |

| | | | | | |
|---|---|---|---|---|---|
| Data is shown as median and interquartile range (IQR). * Significant difference | | | | | |

**Table 8. Quantification of Methanobacteriales present in samples of biopsies of patients (tumor and polyps) and control subjects (healthy). The biopsies included in the analysis correspond to the patient zone.**

| | **Log n° gene copies /mg** | | | | **Mann-Whitney Test** |
|---|---|---|---|---|---|
| **BIOPSIES (n=20)** | **Pr** | **Control (n=5)** | **Pr** | **Patients (n=15)** | **P-value** |
| Methanobacteriales | 5 | 4.38 (4.02-4.39) | 15 | 4.36 (4.14-4.81) | 0.541 |

| | | | | | |
|---|---|---|---|---|---|
| Data is shown as median and interquartile range (IQR). | | | | | |

**Table 9. Quantification of Methanobacteriales present in samples of feces of subjects with polyps and with tumors in comparison with the microbiota present in control subjects (healthy).**

| | **Log n° gene copies /mg** | | | | | | **Kruskal-Wallis Test** |
|---|---|---|---|---|---|---|---|
| | **Pr** | **Control (n=9)** | **Patients (n=15)** | | | | |
| **FECES (n=24)** | | | **Pr** | **polyp (n=8)** | **Pr** | **tumor (n=7)** | **P-value** |
| Methanobacteriales | 9 | 3.43 (3.36-3.63) | 8 | 4.24 (3.34-5.35) | 7 | 6.28 (4.49-6.53)‡ | 0.0314 |

| | | | | | | | |
|---|---|---|---|---|---|---|---|
| Data is shown as median and interquartile range (IQR). ‡ Significant difference (P=0.0033) between feces of subjects with tumors and control subjects using Mann-Whitney U-test | | | | | | | |

**Table 10. Quantification of Methanobacteriales present in samples of biopsies of subjects with polyps and with tumors in comparison with the microbiota present in control subjects (healthy). The biopsies included correspond to the polyps and tumors zone.**

| | **Log n° gene copies /mg** | | | | | | **Kruskal -Wallis Test** |
|---|---|---|---|---|---|---|---|
| | **Pr** | **Control (n=5)** | **Patients (n=15)** | | | | |
| **BIOPSIES (n=20)** | | | **Pr** | **polyp (n=8)** | **Pr** | **tumor (n=7)** | **P-value** |
| Methanobacteriales | 5 | 4.38 (4.02-4.39) | 8 | 4.27 (4.11-4.52) | 7 | 4.87 (4.25-5.02) | 0.340 |

| | | | | | | | |
|---|---|---|---|---|---|---|---|
| Data is shown as median and interquartile range (IQR). | | | | | | | |

**Table 11. Quantification of Methanobacteriales present in samples of biopsies of subjects with polyps and with tumors in comparison with the microbiota present in control subjects (healthy). The biopsies included correspond to the healthy zone.**

| | **Log n° gene copies /mg** | | | | | | **Kruskal-Wallis Test** |
|---|---|---|---|---|---|---|---|
| | **Pr** | **Control (n=5)** | **Patients (n=14)** | | | | |
| **BIOPSIES (n=19)** | | | **Pr** | **polyp (n=8)** | **Pr** | **tumor (n=6)** | **P-value** |
| Methanobacteriales | 5 | 4.38 (4.02-4.39) | 8 | 4.40 (3.79-4.44) | 6 | 4.43 (4.38-4.94) | 0.349 |

| | | | | | | | |
|---|---|---|---|---|---|---|---|
| Data is shown as median and interquartile range (IQR). | | | | | | | |

### Example 10. Mucosal and fecal microbiota composition in health and disease.

Differences in bacterial diversity were observed in mucosal tissue samples (healthy and CRC tissue samples) from the same individual. Furthermore, different microbial profiles were observed between mucosal samples with polyp and adenocarcinoma compared to their corresponding healthy tissue section (Figure 2). Lower abundance of Staphylococcus and Bacillus and higher abundances of Escherichia-Shigella and Prevotella were observed in mucosal samples from polyps compared to the other tissues (Figure 2B), suggesting the potential of those bacterial groups for the diagnosis of CRC development.

The abundance of *Streptococcus* together with *Escherichia-Shigella* was remarkable in polyps and tumors compared to their corresponding healthy tissue as well as with the control group. The above results show the existence of disease biomarkers alternative to the biomarkers disclosed in the state of the art but, as it is mentioned previously, a non-invasive sample like fecal material is more appropriate for developing a diagnostic/prognostic protocol. Although the tests performed showed that fecal samples are not as informative as biopsies, several microorganisms were found to differ between healthy and diseased samples for which fecal material, as well as healthy and diseased tissue samples were available for the same individuals.

In this sense, the adenocarcinomas were classified according to TNM system (Table 1). The inventors have analyzed the impact of tumor severity (T2 and T3) on microbiota composition in order to find a potential biomarker in non-invasive samples. Greater severity of the tumor was related with higher abundance of *Bacteroides* group (0.9% in T2 vs 2.3% in T3, T-test p-value=0.03) and non-significant lower abundances of *Prevotella* (20.2% in T2 vs 7.4% in T3, T-test p-value=0.802) and *Faecalibacterium* (8.7% in T2 vs 6.1% in T3, T-test p-value=0.203) in fecal samples, suggesting those bacterial groups as potential biomarkers of the developmental CRC process, in combination with Archaea group.

Moreover, the impact of tumor severity in mucosal samples was checked and it was observed lower frequency of *Staphylococcus* in advanced tumors (31.5% in T2 vs 3.2% in T3, T-test p-value=0.049). Higher abundance of *Staphylococcus* was detected in T3 mucosal biopsies from healthy tissues compared to adenocarcinoma tissue (14.9% in healthy tissue vs 3.1% in tumor tissue, T-test p-value=0.05). Higher abundance of Staphylococcus was related to lower malignancy and was also associated to healthy tissues (49.46% in T2 healthy tissue; 31.46 % in T2 adenocarcinoma tissue; 14.86 in T3 healthy tissue and 3.12% in T3 adenocarcinoma tissue). Furthermore, in T3 status, it was also found that lower abundance of *Streptococcus* in healthy than in adenocarcinoma tissue (1.67% in healthy tissue vs 8.8% in tumor tissue, T-test P-value=0.09). This trend was also observed in tumor tissues between T2 and T3 groups (1.5% in T2 vs 8.8% in T3, T-test P-value=0.13). Although no significant differences were found in the Escherichia-Shigella group, an interesting trend was detected where increased abundance of this group was related to tumor status (T2 vs T3) and also, with both mucosal tissue samples (0.22% in T2 healthy tissue; 0.97% in T2 adenocarcinoma tissue; 2.85% in T3 healthy tissue and 13.22% in T3 adenocarcinoma tissue).

### Example 11. Diagnosis and/or prognosis of colorectal cancer in a subject.

The following discloses the application of the method of the invention in the diagnosis and/or prognosis of colorectal cancer in a subject. A fecal sample was obtained from a subject H6 showing indication of colorectal cancer. Using quantitative PCR, the number of gene copies of *Methanobacteriales* per mg of sample was determined as 1919335 and the log n° gene copies/mg fecal sample as being 6.28 (cf. **Table 3**)**.**
A) The log n° gene copies of *Methanobacteriales* /mg fecal sample of 6.28 of H6 is greater than the threshold value of log n° gene copies of *Methanobacteriales* /mg fecal sample of 3.65 which was determined from the sampled population of healthy patients (n = 9, mean = 3.43, interquartile range = 3.36-3.63, *cf.* **Table 9**)**,** wherein the threshold value is based on a specificity of more than 75%. In fact, the log n° gene copies of *Methanobacteriales* /mg fecal sample of 6.28 was found to fall outside 3 standard deviations of the mean of the log n° gene copies of *Methanobacteriales* /mg fecal sample of healthy patients *(i.e.* there is less than 1% probability that H6 belongs to the population of healthy patients).
B) The log n° gene copies of *Methanobacteriales* /mg fecal sample of 6.28 of H6 is also greater than the threshold value of log n° gene copies of *Methanobacteriales* /mg fecal sample of 4.45 which was determined from the sampled population of patients with colorectal cancer (n = 7, mean = 6.28, interquartile range = 4.49-6.53, *cf.* **Table 9**)**,** wherein the sensitivity was set to be more than 75%.

Consequently, using either form of comparative analysis, A) or B), the subject H6 was determined to be suffering from colonorectal cancer and was referred to a specialist for confirmation of this result and treatment.

## Claims

1. An *in vitro* method for diagnosis and/or prognosis of colorectal cancer in a subject, using a biological sample obtained from said subject, which comprises:
(a) measuring a level of Archaea microorganism in said biological sample, wherein said Archaea microorganism is a methanogen; and
(b) comparing the level of Archaea microorganism measured in (a) with a reference level of Archaea microorganism in at least one control sample, wherein an elevated level is indicative of colorectal cancer in said subject.

2. The *in vitro* method according to claim 1, wherein the Archaea microorganism is of the methanobacteriales order.

3. The *in vitro* method according to any of claims 1 or 2, wherein the Archaea microorganism is of the methanobrevibacter genus.

4. The *in vitro* method according to any of claims 1 to 3, wherein the level of Archaea microorganism in the biological sample and control sample is measured using primers of any of SEQ ID NO: 40 to SEQ ID NO: 51.

5. The *in vitro* method according to any of claims 1 to 3, wherein the level of Archaea microorganism in the biological sample and control sample is measured using primers of either of SEQ ID NO: 52 or SEQ ID NO: 53.

6. The *in vitro* method according to any of claims 1 to 5 wherein the control sample refers to a population of subjects not having colorectal cancer.

7. The *in vitro* method according to any of claims 1 to 6, wherein the level of Archaea microorganism is measured by real time quantitative polymerase chain reaction.

8. The *in vitro* method according to any of claims 1 to 7, wherein the biological sample is a fecal sample.

9. The *in vitro* method according to any of claims 1 to 8 wherein a level of Archaea microorganism ≥ 3.65 log n° gene copies/mg fecal sample obtained from the subject is indicative of colorectal cancer in said subject.

10. A kit for *in vitro* detection of colorectal cancer in a biological sample obtained from a subject which comprises:
a) a means for measuring a level of Archaea microorganism in said biological sample, wherein said Archaea microorganism is a methanogen; and
b) instructions for comparing the level of Archaea microorganism in said biological sample with a reference level of Archaea microorganism in at least one control sample, wherein an elevated level of Archaea microorganism in said biological sample is indicative of colorectal cancer in said subject.

11. The kit according to claim 10 wherein the means for measuring the level of Archaea microorganism comprises a microarray or a gene chip which comprises commercially available nucleic acid probes, said nucleic acid probes comprising sequences that specifically hybridize to a nucleotide sequence of the Archaea microorganism.

12. The kit according to claim 11 wherein the nucleic acid primers are selected from any of SEQ ID NO: 40 to SEQ ID NO: 51 or from SEQ ID NO: 52 or SEQ ID NO: 53.

13. Use of the method according to any of claims 1 to 9 or the kit according to any of claims 10 to 12 in the *in vitro* diagnosis and/or prognosis of colorectal cancer.
